# EUROPEAN PATENT SPECIFICATION

(11) **EP 1 120 653 B1**
(45) Date of publication and mention of the grant of the patent: **19.10.2005**
(21) Application number: 00946471.0
(22) Date of filing: 24.07.2000
(51) Int. Cl.: G01N 33/68, C12N 15/09, C12Q 1/68, G01N 15/00, G01N 21/78, G01N 33/566, G01N 33/532

(54) **METHOD FOR EXAMINING SUBSTANCE INTERACTING WITH HORMONE RECEPTOR**
VERFAHREN ZUR UNTERSUCHUNG DER WECHSELWIRKUNG VON SUBSTANZ UND HORMONREZEPTOR
PROCEDE VISANT A EXAMINER UNE SUBSTANCE AYANT UNE INTERACTION AVEC UN RECEPTEUR HORMONAL

(30) Priority: 23.07.1999 JP 20986099; 31.05.2000 JP 2000163475; 31.05.2000 JP 2000163476
(43) Date of publication of application: 01.08.2001
(73) Proprietor: Olympus Corporation, Tokyo 151-0072 (JP)
(72) Inventor: SAKAMOTO, Hiroko, Shinagawa-ku, Tokyo 141-0022 (JP); KATO, Noriko, Hachioji-shi, Tokyo 192-0046 (JP)
(74) Representative: Witz, Michael
(86) International application number: PCT/JP2000/004930
(87) International publication number: WO 2001/007919

(56) References cited:
- EP-A2- 0 264 185
- WO-A-00/26366
- WO-A-98/55651
- WO-A1-93/23431
- US-A- 5 846 711
- NELSON S ET AL: "CHARACTERIZATION OF AN INTRINSCALLY FLUORESCENT GONADOTROPIN-RELEASING HORMONE RECEPTOR AND EFFECTS OF LIGAND BINDING ON RECEPTOR LATERAL DIFFUSION" ENDOCRINOLOGY, BALTIMORE, MD, US, vol. 140, no. 2, 1999, pages 950-957, XP002942552 ISSN: 0013-7227
- AWAJI T ET AL: "REAL-TIME OPTICAL MONITORING OF LIGAND-MEDIATED INTERNALIZATION OF ALPHA1B-EDRENOCEPTOR WITH GREEN FLUORESCENT PROTEIN" MOLECULAR ENDOCRINOLOGY, BALTIMORE, MD, US, vol. 12, no. 8, 1998, pages 1099-1111, XP002942553 ISSN: 0888-8809
- T. DRMOTA ET AL: "real time visualization of agonist-mediated redistribution and internalization of a green fluorescent protein-tagged form of the thyrotropin-releasing hormone receptor" JOURNAL OF BIOLOGICAL CHEMISTRY, vol. 273, no. 37, 1998 - 11 September 1998 (1998-09-11), pages 24000-24008, XP002236086
- DAY R N: "VISUALIZATION OF PIT-1 TRANSCRIPTION FACTOR INTERACTIONS IN THE LIVING CELL NUCLEUS BY FLUORESCENCE RESONANCE ENERGY TRANSFER MICROSCOPY" MOLECULAR ENDOCRINOLOGY, BALTIMORE, MD, US, vol. 12, 1998, pages 1410-1419, XP002940052 ISSN: 0888-8809
- WIDENGREN J. ET AL.: 'Fluorescence correlation spectroscopy as a tool to investigate chemical reactions in solutions and cell surfaces' CELLULAR AND MOLECULAR BIOLOGY vol. 44, no. 5, 1998, pages 857 - 879, XP002932538

### SEQUENCE LISTING

<110> OLYMPUS OPTICAL CO., LTD.
<120> Method for testing a substance interacting with hormone receptor.
<130> 00S0496P
<150> JP/11-209860
   <151> 1999-07-23
<150> JP/2000-163475
   <151> 2000-05-31
<150> JP/2000-163476
   <151> 2000-05-31
<160> 3
<170> PatentIn Ver. 2.0
<210> 1
   <211> 1740
   <212> DNA
   <213> Homo sapiens
<220>
   <221> CDS
   <222> (99)..(1688)
<400> 1
   gttgacagcc attatacttg cccacgaatc tttgagaaca ttataatgac ctttgtgcct 60
   cttcttgcaa ggtgttttct cagctgttat ctcaagac atg gat ata aaa aac tca 116
                                             Met Asp Ile Lys Asn Ser
                                              1               5
   cca tct agc ctt aat tct cct tcc tcc tac aac tgc agt caa tcc atc 164
   Pro Ser Ser Leu Asn Ser Pro Ser Ser Tyr Asn Cys Ser Gln Ser Ile
   10 15 20
   tta ccc ctg gag cac ggc tcc ata tac ata cct tcc tcc tat gta gac 212
   Leu Pro Leu Glu His Gly Ser Ile Tyr Ile Pro Ser Ser Tyr Val Asp
   25 30 35
   agc cac cat gaa tat cca gcc atg aca ttc tat agc cct gct gtg atg 260
   Ser His His Glu Tyr Pro Ala Met Thr Phe Tyr Ser Pro Ala Val Met
   40 45 50
   aat tac agc att ccc agc aat gtc act aac ttg gaa ggt ggg cct ggt 308
   Asn Tyr Ser Ile Pro Ser Asn Val Thr Asn Leu Glu Gly Gly Pro Gly
   55 60 65 70
   cgg cag acc aca agc cca aat gtg ttg tgg cca aca cct ggg cac ctt 356
   Arg Gln Thr Thr Ser Pro Asn Val Leu Trp Pro Thr Pro Gly His Leu
   75 80 85
   tct cct tta gtg gtc cat cgc cag tta tca cat ctg tat gcg gaa cct    404
   Ser Pro Leu Val Val His Arg Gln Leu Ser His Leu Tyr Ala Glu Pro
                   90                  95                  100
   caa aag agt ccc tgg tgt gaa gca aga tcg cta gaa cac acc tta cct    452
   Gln Lys Ser Pro Trp Cys Glu Ala Arg Ser Leu Glu His Thr Leu Pro
               105                 110                 115
   gta aac aga gag aca ctg aaa agg aag gtt agt ggg aac cgt tgc gcc    500
   Val Asn Arg Glu Thr Leu Lys Arg Lys Val Ser Gly Asn Arg Cys Ala
           120                 125                 130
   agc cct gtt act ggt cca ggt tca aag agg gat gct cac ttc tgc gct    548
   Ser Pro Val Thr Gly Pro Gly Ser Lys Arg Asp Ala His Phe Cys Ala
   135                 140                 145                 150
   gtc tgc agc gat tac gca tcg gga tat cac tat gga gtc tgg tcg tgt    596
   Val Cys Ser Asp Tyr Ala Ser Gly Tyr His Tyr Gly Val Trp Ser Cys
                   155                 160                 165
   gaa gga tgt aag gcc ttt ttt aaa aga agc att caa gga cat aat gat    644
   Glu Gly Cys Lys Ala Phe Phe Lys Arg Ser Ile Gln Gly His Asn Asp
                   170                 175                 180
   tat att tgt cca gct aca aat cag tgt aca atc gat aaa aac cgg cgc    692
   Tyr Ile Cys Pro Ala Thr Asn Gln Cys Thr Ile Asp Lys Asn Arg Arg
               185                 190                 195
   aag agc tgc cag gcc tgc cga ctt cgg aag tgt tac gaa gtg gga atg 740
   Lys Ser Cys Gln Ala Cys Arg Leu Arg Lys Cys Tyr Glu Val Gly Met
   200 205 210
   gtg aag tgt ggc tcc cgg aga gag aga tgt ggg tac cgc ctt gtg cgg 788
   Val Lys Cys Gly Ser Arg Arg Glu Arg Cys Gly Tyr Arg Leu Val Arg
   215 220 225 230
   aga cag aga agt gcc gac gag cag ctg cac tgt gcc ggc aag gcc aag 836
   Arg Gln Arg Ser Ala Asp Glu Gln Leu His Cys Ala Gly Lys Ala Lys
   235 240 245
   aga agt ggc ggc cac gcg ccc cga gtg cgg gag ctg ctg ctg gac gcc 884
   Arg Ser Gly Gly His Ala Pro Arg Val Arg Glu Leu Leu Leu Asp Ala
   250 255 260
   ctg agc ccc gag cag cta gtg ctc acc ctc ctg gag gct gag ccg ccc 932
   Leu Ser Pro Glu Gln Leu Val Leu Thr Leu Leu Glu Ala Glu Pro Pro
   265 270 275
   cat gtg ctg atc agc cgc ccc agt gcg ccc ttc acc gag gcc tcc atg 980
   His Val Leu Ile Ser Arg Pro Ser Ala Pro Phe Thr Glu Ala Ser Met
   280 285 290
   atg atg tcc ctg acc aag ttg gcc gac aag gag ttg gta cac atg atc 1028
   Met Met Ser Leu Thr Lys Leu Ala Asp Lys Glu Leu Val His Met Ile
   295 300 305 310
   agc tgg gcc aag aag att ccc ggc ttt gtg gag ctc agc ctg ttc gac 1076
   Ser Trp Ala Lys Lys Ile Pro Gly Phe Val Glu Leu Ser Leu Phe Asp
   315 320 325
   caa gtg cgg ctc ttg gag agc tgt tgg atg gag gtg tta atg atg ggg 1124
   Gln Val Arg Leu Leu Glu Ser Cys Trp Met Glu Val Leu Met Met Gly
   330 335 340
   ctg atg tgg cgc tca att gac cac ccc ggc aag ctc atc ttt gct cca 1172
   Leu Met Trp Arg Ser Ile Asp His Pro Gly Lys Leu Ile Phe Ala Pro
   345 350 355
   gat ctt gtt ctg gac agg gat gag ggg aaa tgc gta gaa gga att ctg 1220
   Asp Leu Val Leu Asp Arg Asp Glu Gly Lys Cys Val Glu Gly Ile Leu
   360 365 370
   gaa atc ttt gac atg ctc ctg gca act act tca agg ttt cga gag tta 1268
   Glu Ile Phe Asp Met Leu Leu Ala Thr Thr Ser Arg Phe Arg Glu Leu
   375 380 385 390
   aaa ctc caa cac aaa gaa tat ctc tgt gtc aag gcc atg atc ctg ctc 1316
   Lys Leu Gln His Lys Glu Tyr Leu Cys Val Lys Ala Met Ile Leu Leu
   395 400 405
   aat tcc agt atg tac cct ctg gtc aca gcg acc cag gat gct gac agc 1364
   Asn Ser Ser Met Tyr Pro Leu Val Thr Ala Thr Gln Asp Ala Asp Ser
   410 415 420
   agc cgg aag ctg gct cac ttg ctg aac gcc gtg acc gat gct ttg gtt 1412
   Ser Arg Lys Leu Ala His Leu Leu Asn Ala Val Thr Asp Ala Leu Val
   425 430 435
   tgg gtg att gcc aag agc ggc atc tcc tcc cag cag caa tcc atg cgc 1460
   Trp Val Ile Ala Lys Ser Gly Ile Ser Ser Gln Gln Gln Ser Met Arg
   440 445 450
   ctg gct aac ctc ctg atg ctc ctg tcc cac gtc agg cat gcg agt aac 1508
   Leu Ala Asn Leu Leu Met Leu Leu Ser His Val Arg His Ala Ser Asn
   455 460 465 470
   aag ggc atg gaa cat ctg ctc aac atg aag tgc aaa aat gtg gtc cca 1556
   Lys Gly Met Glu His Leu Leu Asn Met Lys Cys Lys Asn Val Val Pro
   475 480 485
   gtg tat gac ctg ctg ctg gag atg ctg aat gcc cac gtg ctt cgc ggg 1604
   Val Tyr Asp Leu Leu Leu Glu Met Leu Asn Ala His Val Leu Arg Gly
   490 495 500
   tgc aag tcc tcc atc acg ggg tcc gag tgc agc ccg gca gag gac agt 1652
   Cys Lys Ser Ser Ile Thr Gly Ser Glu Cys Ser Pro Ala Glu Asp Ser
   505 510 515
   aaa agc aaa gag ggc tcc cag aac cca cag tct cag tgacgcctgg 1698
   Lys Ser Lys Glu Gly Ser Gln Asn Pro Gln Ser Gln
   520 525 530
   ccctgaggtg aactggccca cagaggtcac aagctgaagc gt 1740

## Description

### Technical Field

The present invention relates to a method for testing a substance (e.g., endocrine disrupting chemicals which are also called an "environmental hormone"), which interacts with a hormone receptor protein.

### Background Art

Recently, studies have been aggressively carried out on the effects of environmental chemical substances upon the endocrine system in an organism. In particular, the subject on endocrine disrupting chemicals is an important area of study. Endocrine disrupting chemicals, also known as an "environmental hormone", are defined as an exogenous chemical substance or a mixture thereof which affect the function of the endocrine system, thereby inducing a harmful effect upon individuals, offsprings or a population (sub population). The mechanism of action of the endocrine disrupting chemicals is recognized as follows. When the endocrine disrupting chemicals are bound to a hormone receptor present in a living body, it prevents a natural hormone from binding to the hormone receptor, or a false hormonal signal is inappropriately emitted, which may adversely affect in vivo. Recently, it has become known that the endocrine disrupting chemicals disrupt the endocrine systems even in low concentrations, at which the endocrine disrupting chemicals cannot be detected in conventional toxicity tests using animals, However, it is still unknown whether a single endocrine disrupting chemicals produces a harmful effect due to long-term exposure or a multiple combination of a wide variety of endocrine disrupting chemicals produce the harmful effects.
Thus, the mechanism of action of the endocrine disrupting chemicals has not yet been elucidated.

In the background of the reasons why the mechanism has not yet been made clear, the following facts are present. A traditional toxicity test using animals (which is directed to detecting a lethal toxicity and chronic damage to organs) employs, as an indicator, the damage and lethal toxicity caused by administration of a single endocrine disrupting chemical. Therefore, it is impossible to detect the substance affecting the endocrine system, etc. in low concentrations. Under the circumstances, internationally, most notably in OECD, guidelines on a screening test of endocrine disrupting chemicals have been started drawing up. However, the guidelines have not been fully established yet.

It is known that the chemical substances having an endocrine disrupting activity mostly have a function similar to that of estrogen (which is female hormone). Therefore, in methods for detecting the endocrine disrupting chemicals, most methods have been developed with the aim of detecting the estrogen-like activity of the chemical substances. It is considered that these chemical substances may cause the endocrine disrupting activity by binding to estrogen receptors.

Typical methods for detecting endocrine disrupting chemicals having an estrogen-like activity are as follows.

### ① Receptor binding assay

In this method, the endocrine disrupting activity is detected by adding a chemical substance to be tested (hereinafter referred to as "test substance") and radioisotope-labeled β-estradiol (one of estrogens) simultaneously to purified estrogen receptors and determining the difference in receptor binding ability between them.

This method is advantageous in that no cells are required, and that the endocrine disrupting activity of a test substance can be checked within 24 hours. In addition, this method has an excellent level of sensitivity. However, this method is disadvantageous in that it is needed to use radioisotope. Another disadvantage resides in that the method is only directed to the binding ability of the test substance to the receptor, so that it fails to evaluate whether the substance can actually induce the physiological activity in vivo. Therefore, it fails to evaluate that the physiological activity results from either agonist activity or antagonist activity. To overcome the problem with the use of a radioisotope, a fluorescence-labeled estrogen(β-estradiol) has been recently employed in the detection method. However, it is now impossible to evaluate that the physiological activity results from either the agonist activity or the antagonist activity, and this problem still remains unsolved. Evaluation of a specific physiological activity of a test substance in vivo is important in evaluating the pharmacological efficacy of a chemical substance such as a synthetic hormone, for developing a pharmacological agent using the synthetic hormone. Therefore, it has been strongly desired to develop an assay capable of evaluating whether the physiological activity of the test substance results from either the agonist activity or the antagonist activity.

### ② Reporter gene assay

In this method, a test substance is added to cultured cells into which a reporter gene having an estrogen responsive element has been transfected.
Then, the endocrine disrupting activity induced by the test substance is detected by quantifying the amount of the final product produced by the endocrine disrupting activity, that is, the product of the reporter gene.

This method takes several days to determine the endocrine disrupting activity of a test substance. The period is longer than that of the receptor binding assay ①. As the cells, cultured cells derived from yeast or humans may be used. As the reporter gene, generally β-galactosidase or the like may be used. Furthermore, this method requires a specific reagent (e.g., a luminescent substrate in the case where the reporter gene is β-galactosidase) for quantifying the product of the reporter gene. In addition, this method employs, as a detection indicator, the presence or absence of the product of the reporter gene, which is produced as a result of the endocrine disrupting activity. Therefore, it is possible to evaluate the agonist activity between the two physiological activities (i.e., the agonist and the antagonist activity) of the test substance in vivo. However, it is difficult to evaluate the antagonist activity.
In particular, if yeast cells are used, this assay can be performed easily owing to bacterial cells. However, yeast cells have a cell wall, which lowers the permeability of the test substance into the cell. Due to this, the detection sensitivity may significantly decrease. This is a serious disadvantage.

### ③ Proliferative acceleration test

In this method, a test substance is added to cultured cells expressing estrogen receptor, and whether or not the proliferation of cells is accelerated is detected.

This method requires about one week to detect the endocrine disrupting activity of a test substance. The detection time is longer than that of the reporter gene assay ②. In this method, after a test substance is added to cultured cells expressing estrogen receptor, such as MCF-7 or ISHIKAWA, the proliferation of the cells is observed. The observation is compared with those of a control group to which β-estradiol is added. Whether the test substance has the endocrine disrupting activity is determined by the extent to which the proliferation of the cells is accelerated in the presence of the test substance. This method is simple in that a specific reagent is not required, unlike the methods ① and ②. However, this method requires a considerably large amount of cells compared to the reporter gene assay ②. Time and effort are required to prepare the cells. In this method, as in the reporter gene assay ②, it is difficult to evaluate the antagonist activity among the physiological activities (i.e., agonist and antagonist activity) of the chemical substance in Vivo. Furthermore, if the test substance exhibits a proliferative activity (e.g., the test substance is a growth factor, carcinogenic promoter and the like), a false positive (exhibiting positive) may likely result.

### ④ Test of animal individual

The test systems of ① to ③ have disadvantages in that pharmacokinetics (absorption, distribution, metabolism, excretion) is not reproducible. On the other hand, in the test of animal individual, an internal environment (an environment in a living body) can be more appropriately reproduced. The test of animal individual includes a uterus proliferative assay and a Hershberger assay using rodents and amphibians. However, each assay has the following problems: detection takes up to several weeks. Extra equipment and a lot of cost are required for raising animals.
The test of animal individual leads to sacrifice to animals. Furthermore, the functional molecule that interacts with a test substance varies depending upon the animal species. Therefore, it is sometimes difficult to extrapolate the obtained results to humans.

Under the circumstances, it has been desired to develop a highly-sensitive and specific detection method for endocrine disrupting chemicals, whereby the aforementioned problems associated with the test methods ① to ④ are resolved. Nevertheless, a satisfactory method has not yet been obtained until now. WO-A-00/26366 discloses cell-based assays for detecting an analyte based on the use of cells expressing a fusion protein obtained by fusing a surface cell estrogen receptor with a fluorescent protein. The presence of a receptor's ligand is detected by a change in fluorescence in the sample. S. Nelson et al (1999) Endocrinol. 140, 950-957, T. Awaji et al (1998) Mol. Endocrinol. 12, 1099-1111 and T. Drmota et al (1998) J. Biol. Chem. 273, 24000-24008 disclose the use of fusion proteins obtained by fusing a hormone receptor protein with a fluorescent protein for monitoring ligand binding and internalization in living cells. R. N. Day (1998) Mol. Endocrinol. 12, 1410- 1418 discloses a method for visualization of Pit-1 transcription factor interactions in living cells based on measurements of the fluorescence resonance energy transfer upon binding of Pit-1 fused to green fluorescent protein to an estrogen receptor fused to blue fluorescent protein.

The method for detecting an endocrine disrupting chemicals must satisfy all of the requirements: detecting a very small amount of substances; having an analogous mechanism to the human body; and being simple in terms of equipment, cost, test period, etc.

The estrogen receptor is a protein belonging to a nuclear hormone receptor superfamily. In other words, the estrogen receptor is a ligand-inducible transcriptional regulatory factor which controls the expression of a target gene by binding a ligand such as estrogen hormone.

AS the estrogen receptor, only an estrogen receptor α (ERα) consisting of 595 amino acids has been known. However, in 1996, a sub-type of the estrogen receptor consisting of 530 amino acids was found, which has a high homology with ERα and can bind estrogen as a ligand. This is designated as estrogen receptor β (ERβ). The gene of ERβ has been isolated.

Both type of estrogen receptors have analogous structures. The structure consist of four functional domains: an A/B region (Activation Function-1: AF-1) on the N terminus side; an E/F region (Activation Function-2: AF-2) on the C terminus side, both having a transcriptional activation ability; and a C region (DNA binding domain: DBD) having two Zinc finger motifs and having a DNA binding ability; and a D region having a signal (NLS) for use in transferring an estrogen receptor itself into a nucleus. The main region for the hormone receptor, namely, a ligand-binding region, resides in the E/F region.

The estrogen receptor α (hereinafter referred to as "ERα") and the estrogen receptor β (hereinafter referred to as "ERβ") differ in only three amino acids in their DNA binding regions, so that they have a high homology. In contrast, they have only 59% homology in the ligand binding regions. This fact suggests that the two types of the estrogen receptors independently recognize different ligand, respectively.

Furthermore, ERα and ERβ are reported to be expressed in different tissues. It is also reported that their expression period differs, even if they are expressed in the same tissue. Based on these findings, it is postulated that ERα and ERβ are responsible for different biological function.

There was an interest finding in a recent report (J-Å Gustafsson, Estrogen receptor β - a new dimension in estrogen mechanism of action. J. Endocrinology 163, 379-383 (1999), which showed that ERβ was found to be highly expressed in a testis or an ovary (which is susceptible to endocrine disrupting chemicals). In the testis, ERβ is expressed particularly in a sperematogonium and a sperematocyte. This finding suggests that ERβ plays an important role in spermatogenesis. More specifically, it is postulated that a decrease in the number of spermatozoa and spermatozoa-dysfunction due to endocrine disrupting chemicals, may be phenomena mediated by ERβ rather than ERα.

As mentioned above, it is conceivable that many endocrine disrupting chemicals may have a significant function via ERβ besides ERα. Under the circumstances, it has been desired to develop a system for efficiently detecting a substance which targets ERβ.

On the other hand, an MCF-7 strain is generally used as the cultured cells in the detection system of endocrine disrupting chemicals mentioned above. Unfortunately, the MCF-7 cells express ERα at a high rate. Therefore, a substance specifically binding to ERβ cannot be detected at present.

Furthermore, in the field of drug design, the compound which targets an estrogen receptor is screened. At that time, if a compound which targets ERα is screened independently of a compound which targets ERβ, the compound which functions tissue-specifically can be screened. This leads to development of a hormonal drug exhibiting low side effects. Thus, it is very useful to efficiently detect a substance which targets ERβ.

Therefore, the present invention is attained in order to resolve the aforementioned problems and to test the substance interacting with a hormone receptor protein. To attain the objects, the present invention aims at construction of a simple evaluation system analogous to a living body, and capable of detecting the behavior of very small amount of molecules.

### Disclosure of Invention

A method for testing a substance interacting with a hormone receptor according to the present invention is characterized by the features of the appended claims.

### Brief Description of Drawings

FIG. 1 is a schematic view showing a fusion gene expression plasmid.
FIG. 2 is a micrograph showing a cultured cell line transfected with GERb3-9-1 plasmid.
FIG. 3 is an illustration of a strategy for detecting estrogen-like endocrine disrupting chemicals.
FIGS. 4A and 4B are graphs showing characteristics of the excitation wavelength and luminescence wavelength of the fluorescent proteins, respectively.
FIG. 5 is a schematic diagram showing means for screening the cells suitable for detection by FCS.
FIG. 6 is a diagram showing the results of FCS measurement varied by the amount of fluorescence.
FIG. 7 is a graph showing an autocorrelation function when the cells having appropriate fluorescence intensity are measured.
FIG. 8 is a schematic view of the inside of a cell during FCS measurement.
FIG. 9 is diagram showing a part of the nucleotide sequence of a fusion gene.
FIG. 10 is a graph showing the results analyzed by flow cytometry.
FIG. 11 is a graph showing average fluorescence intensity in individual clones of a GFP-gene transferred cell.
FIG. 12 is a graph showing average fluorescence intensity in individual clones of a YFP-gene transferred cell.
FIG. 13 is a graph showing average fluorescence intensity in individual clones of a CFP-gene transferred cell.
FIG. 14 is a graph showing the results obtained by FCS measurement immediately after (0 minutes) and 45 minutes after the addition of 17β estradiol.

### Best Mode for Carrying Out of the Invention

In the method for testing an endocrine disrupting chemicals in a living cell according to the present invention, a receptor is labeled with a marker substance, and a ligand and a test substance are not labeled.

As the hormone receptor used in the present invention, any hormone receptor may be used as long as it causes a change of the state by binding its natural ligand, leading to a change in detectable characteristics of the optical signal emitted from the marker substance. The hormone receptor may be a receptor positioning on a cell membrane, a receptor present inside a cell, or an intranuclear receptor. Of them, the receptor present inside a cell is preferable, and the intranuclear receptor is more preferable. Examples of preferable hormone receptors are receptors belonging to a nuclear hormone receptor superfamily, e.g., an estrogen receptor, a progesterone receptor, a thyroid hormone receptor, and a glucocorticoid receptor. The particularly preferable receptor protein is an estrogen receptor protein, which is conventionally used for detecting endocrine disrupting chemicals. Of the estrogen receptor, a human estrogen receptor β (hereinafter referred to as "hERβ") is the most preferable. This is because it has been desired to detect a substance causing an endocrine disrupting activity via hERβ, as set forth in the paragraph of Prior Art.

In the present invention, any substance may be employed as the marker substance for labeling the hormone receptor protein as long as it emits a detectable optical signal. Either a chemiluminescent substance or a fluorescent substance may be used. However, in order to investigate a change in state of the intracellular hormone receptor protein while a cell is maintained alive, a preferable marker substance is one which is nontoxic to the cell and emits light in absence of a substrate. Particularly a fluorescent protein is preferable. Examples of the fluorescent proteins include GFP (green fluorescent protein), CFP (cyan fluorescent protein), YFP (yellow fluorescent protein) and RFP (red fluorescent protein). These fluorescent proteins are fused with a hormone receptor protein, and the resultant fusion protein can be expressed by a well-known recombinant gene technology, as will be described later.

In the present invention, changes in the state of the hormone receptor protein include, as an example, a change in the apparent molecular weight of the hormone receptor. To give an example, the change in apparent molecular weight is induced by dimerization of the hormone receptor protein/test substance complex. This state-change may include not only a state-change in a single-stage but also state-changes in plurality of stages. Each of these state-changes constitutes a signal transduction pathway for expressing the physiological activity of a hormone.

A change in a detectable characteristic of the optical signal emitted from the marker substance may include a change in wavelength of the optical signal to be detected, a change in intracellular distribution (localization) of the optical signal to be detected, and a change in the fluorescence intensity to be detected by a fluorescence correlation spectroscopy (hereinafter simply referred to as "FCS"). However, the means for capturing a change in the detectable characteristic of the optical signal may not be limited to these. In any event, the change in the detectable characteristic of the optical signal must be attributed to a state-changes caused by binding its natural ligand to the hormone receptor.

Note that FCS is a technique for accurately measuring microscopic movements of individual target molecules. More specifically, the FCS is performed by measuring the fluctuation movement of the target molecule labeled with fluorescence in a medium and then analyzing the measurement results by an autocorrelation function (reference: D. Magde and E. Elson, "Fluorescence correlation spectroscopy II.
An experimental realization", Biopolymers 13(1) 29-61). The principle of FCS applied in the present invention will be explained as follows: In FCS, a fluorescent signal emitting from a micro field of vision in a sample is quantified by a microscopy. Since the target molecules labeled with fluorescence are moving around in a medium, the quantified value of the fluorescence intensity varies depending upon how frequently the target molecules come into the micro field of vision and how long the target molecules stay in the micro field. To be more specific, if the apparent molecular weight increases due to the dimerization, the movement of the target molecules slows down, and the apparent number of the molecules decreases. It follows that the frequency of the target molecules coming into the micro field of vision decreases, with the result that the fluorescence intensity detected changes. Therefore, by measuring the change in the fluorescence intensity, the change in apparent molecular weight of the target molecule can be monitored.

Since molecular fluctuation in the micro field can be detected by FCS, the FCS analysis is useful for specifically detecting intermolecular interaction with a high sensitivity. More specifically, the FCS analysis is performed as follows. First, the Brownian movement of fluorescent molecules present in a solution is detected in a micro field by a confocal laser microscope. Then, the diffusion time is analyzed based on the fluctuation of the fluorescence intensity.
Based on the diffusion time, a physical amount such as number and size of the molecules is determined.

To measure the movement of intracellular organic molecules by FCS, the target organic molecules must be labeled with a marker substance emitting fluorescence while maintaining the function of the cells as it is. Examples of the conventional marker substances emitting fluorescence include FITC, rhodamine, Cy3 (available from Amarsham Pharmacia), Cy5 (available from Amarsham Pharmacia), and PI (Propydium Iodide). However, most of these marker substances are cytotoxic. Due to the cytotoxicity, it has been difficult to measure the movement of the intracellular organic molecules by FCS.

Biological applications of FCS are reported in documents (Cell Mol. Biol. 44(5), 857-879 (1998); Biochemistry, 37, 12971-12978 (1998)). However, nobody has disclosed the invention which is specialized in screening a substance interacting with the hormone receptor (e.g., endocrine disrupting chemicals), by applying FCS to an intracellular hormone receptor labeled with fluorescence in order to analyze the hormone receptor.

In the present invention, the predetermined conditions refer to the conditions in which a natural ligand can bind to the hormone receptor and cause a change of the state as mentioned above. The predetermined conditions may be either inside the cultured cell or in the solution contained in a test chamber. When the cultured cell is employed as a test system, human cultured cells such as MCF-7 and HeLa, as well as non-human mammalian cells including CHO cells derived from a Chinese hamster, may be used.

In the present invention, the test substance refers to any chemical substance except a natural ligand to the hormone receptor. Therefore, any substance suspected of having an endocrine disrupting activity, may be used.

In the present invention, after a test substance is added, the predetermined conditions is maintained, thereby the test substance is bound to the hormone receptor, if the test substance is an endocrine disrupting factor responsive to the hormone receptor. As a result, the same series of biochemical reactions as that caused by binding the natural ligand to the receptor, take place. At that time, the state of the receptor changes, and thereby the optical signal emitting from the marker substance also changes. Therefore, by detecting the change of the optical signal, it is possible to know whether or not the test substance corresponds to endocrine disrupting chemicals. If no change occurs in the optical signal before and after the addition of the test substance, the test substance does not interact with the hormone receptor, showing that the test substance dose not correspond to an endocrine disrupting chemicals to the hormone receptor.

In a preferable embodiment of the present invention, the hormone receptor labeled with fluorescence is made to express inside a cell, in order to construct a mechanism analogous to a living body. In this respect, the present invention greatly differs from the conventional case where the ligand is labeled.

Specifically, the preferable embodiment of the present invention is as follow: ① A recombinant gene is prepared by combining a gene encoding the hormone receptor protein present in a living body with a gene encoding a marker substance capable of emitting an optical signal. This preparation is performed by constructing an expression vector (hereinafter also referred to as expression plasmid) containing the recombinant gene (i.e., the fusion gene). Subsequently, ② the expression vector containing the fusion gene is introduced into a cultured cell, so that the fusion protein comprising the hormone receptor and the marker substance emitting an optical signal is allowed to express inside the cell. Thereafter, ③ the test substance is added to the cell expressing the fusion protein comprising the hormone receptor and the marker substance. The intracellular signal transduction via the fusion protein is induced by the addition of the test substance. The signal transduction is detected by measuring the optical signal for a predetermined period by focusing in an inner region of the cell by means of FCS or a fluorescence microscope. In this manner, it is possible to detect the interaction between the test substance and the receptor molecule. Based on this, the test substance is evaluated for the endocrine disrupting activity. The phrase an inner region of the cell" may be the entire region inside the cell or a part of the intracellular region. As the part of the intracellular region, any intracellular region may be used. When the measurement is performed by FCS, it follows that a specific region inside the cell is measured.

In the following, details of the above ① to ③ will be given in order.

### ① Preparation of the fusion gene and construction of the expression plasmid

As previously mentioned, the gene encoding a hormone receptor protein used in the present invention is not restricted as long as it can be detected, as a detectable change, an optical signal emitted when a natural ligand is bound to the receptor protein, compared with the optical signal emitted from the receptor protein before binding the ligand. Furthermore, as previously mentioned, since it has been desired to detect a substance causing an endocrine disrupting activity via human estrogen receptor β (hereinafter, also referred to as "hERβ), the hERβ gene is most preferably used. However, the gene encoding the hormone receptor protein is not limited to the hERβ gene in the present invention.

In the present invention, as the gene encoding the hormone receptor protein, any gene may be used as long as it contains at least a coding region having a code for the amino acid sequence of the hormone receptor protein, in other words, a gene sequence from the initiation codon to the termination codon. More specifically, the gene may have any length of a sequence upstream of the initiation codon and a sequence downstream of the termination codon, as long as it does not adversely affect the expression of the hormone receptor protein,

For example, it is sufficient that the human estrogen receptor β gene (sequence of cDNA is represented by Sequence ID No. 1) may contain the nucleotide sequence of the coding region from the 99th to 1688th position. The fusion gene actually prepared in the present invention has the sequence of the 53th to 1735th position of the hERβ gene, as shown in examples described later. However, the length of the sequence of the hERβ gene in the fusion gene varies depending upon cloning conditions of the hERβ gene and preparation conditions of the fusion gene. Therefore, the length of the sequence of the hERβ gene should not be limited to those prepared in the examples below.

The gene encoding the marker substance to be fused with the hormone receptor gene is not particularly limited as long as it is a gene of a substance capable of emitting a detectable optical signal. As the gene encoding the marker substance, a gene encoding either a chemiluminescence substance or a substance emitting fluorescence may be used. Furthermore, the gene encoding the marker substance may have any length of a sequence, as long as the gene contains the coding region of the marker substance and can express the marker substance.

As the gene for the marker substance, a gene of the fluorescent protein is preferable. Examples of the gene coding the fluorescent protein include the genes of GFP (green fluorescent protein), CFP (cyan fluorescent protein), and YFP (yellow fluorescent protein). These genes of fluorescent proteins are commercially available in the form of being introduced into expression vectors. It is desirable to use the commercially available genes of the fluorescent proteins in consideration of the preparation and expression of the fusion gene.

The gene encoding a hormone receptor is fused with the gene encoding a marker substance as follows: First, a desired hormone receptor gene is isolated by cloning. Then, the hormone receptor gene thus prepared is ligated to the marker substance gene which has been previously introduced in an expression vector.

For the ligation, the sequence of the ligation portion of the marker substance gene and the receptor gene includes a sequence of any multi-cloning site.
The sequence of the multi-cloning site is not particularly limited, as long as it enables cleavage with restriction enzymes and ligation with ligase. Further, it is not restricted as long as it has the length to the extent of which it does not adversely affect the expression of the fusion protein.

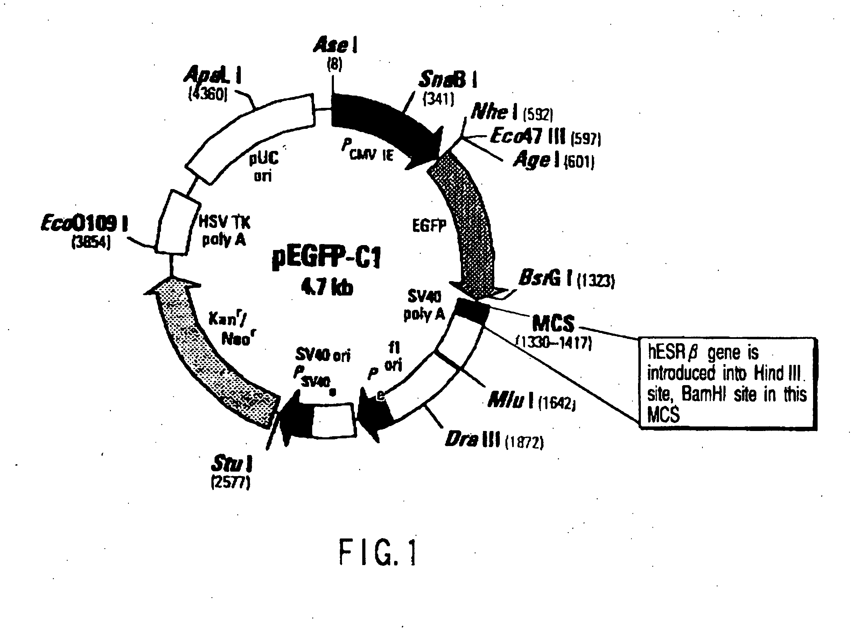

It is convenient to use a commercially available expression vector wherein the marker substance gene is previously introduced. FIG. 1 shows an example of the aforementioned expression vector, pEGFP-C1. This expression vector has a gene coding green fluorescent protein as the marker substance gene (EGFP in the figure) and also includes the multi-cloning site (MCS in the figure) downstream of the EGFP gene. The fusion gene with the EGFP gene can be prepared by inserting any gene into the MCS. Furthermore, the fusion gene can be expressed with high frequency in a mammalian cultured cell under the regulation of the promoter (^{P}CMV IE in the figure), which is located upstream thereof. The vector includes a drug resistant marker gene (Kan^{r}/Neo^{r}) which may be used for screening the cell containing the vector therein. Further, the multi-cloning site present in the expression vector serves as the joint, when the marker substance gene, which is previously introduced into the expression vector, is ligated with the receptor gene.

As described above, by using a desired expression vector containing the marker substance gene, it is possible to prepare the fusion gene and to construct the expression plasmid.

### ② Transfection of a cell with the expression plasmid and expression of the fusion protein

A cell is transfected with the expression plasmid containing the fusion gene constructed above. It is preferable that a human cultured cell be used for the transfection in order to have a purpose of evaluating the endocrine disrupting activity in humans. As an example of the human cultured cell, a cell strain derived from human mammary cancer tissue may be mentioned. The transfection can be performed in accordance with a known method such as a lipofection method, a calcium phosphate method, or an electroporation method. However, the electroporation method (described in examples later) is used preferably, since a plasmid DNA can be efficiently introduced.

Thereafter, drug resistant transfected cells are screened from the cells thus transfected. In this manner, a stable transfected cell line can be established. In the present invention, as mentioned above, it is preferable that the transfected cells be screened by using the expression plasmid containing the drug resistant marker gene.

The aforementioned transfected cells express the fusion gene under the regulation of the promoter in the expression plasmid. The fusion protein expressed can emit an optical signal while maintaining the function of the hormone receptor. As long as the protein satisfies the aforementioned conditions, a single or a few amino acids may be deleted from the amino acid sequence of the protein, a single or a few amino acids of the protein may be displaced, or a single or a few amino acids may be added to the amino acid sequence of the protein.

### ③ Detection of endocrine disrupting activity of a test substance mediated by a hormone receptor

When a test substance is added to a cell expressing the fusion protein of the hormone receptor and the marker substance or added to the purified fusion protein, the state-change of the hormone receptor protein may be induced subsequently. The state-change is detected by FCS or an optical microscope. In this manner, the endocrine disrupting activity of the test substance can be evaluated.

In the following, we will explain a preferable embodiment of the present invention, i.e., a preferable case where the endocrine disrupting activity of the test substance is evaluated by adding the test substance to the cell expressing the fusion protein of the estrogen receptor and the marker substance or adding to the purified fusion protein.

First, the estrogen receptor itself and the mechanism of action thereof will be explained.

As mentioned above, the estrogen receptor is a protein belonging to an intranuclear hormone receptor superfamily, and also known as a ligand-inducible transcriptional regulatory factor, which is responsible for regulating the expression of a target gene by binding a ligand such as estrogen.

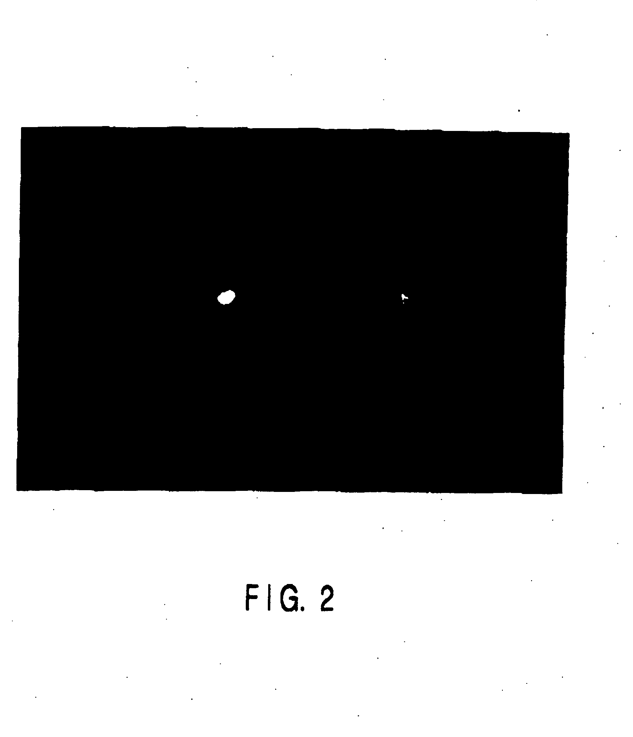

The estrogen receptor protein, after being produced in the cytoplasm, swiftly moves into the nucleus and usually localizes in the nucleus. The localization of the estrogen receptor protein in a nucleus is also demonstrated in the present invention, as shown in FIG. 2.

When a ligand (e.g., estrogen) passes through the cell membrane, comes in inside a cell, and binds to the estrogen receptor, the two receptors form the dimer. The dimer recognizes a specific nucleotide sequence of intranuclear DNA (estrogen responsive element sequence: ERE), thereby forming a complex of receptor/DNA. The complex of receptor/DNA is activated by a coactivator present in the nucleus, thereby accelerating transcription of a gene located downstream of ERE.

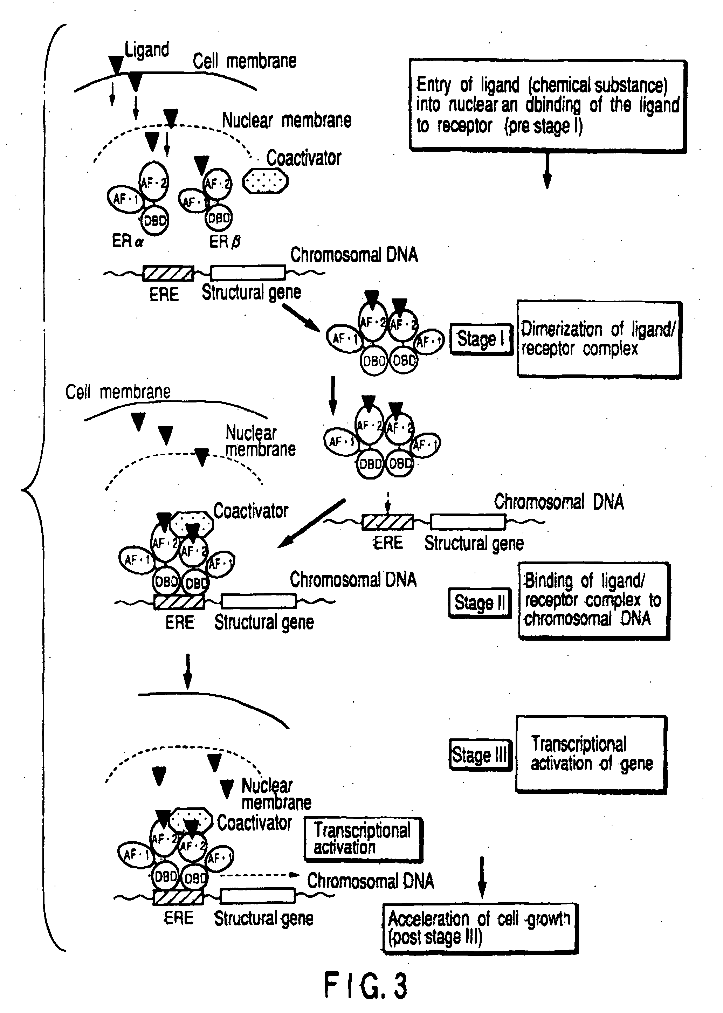

In consideration of the action-mechanism of the estrogen receptor mentioned above, we had the idea that the estrogen-like endocrine disrupting chemicals can be detected in the following three stages, as shown in FIG. 3.
(1) Stage I where the estrogen-like endocrine disrupting chemicals is bound to an estrogen receptor (hereinafter, referred to as "ER"), thereby forming an ER dimer.
(2) Stage II where the ER dimer binds to an estrogen responsive element sequence (hereinafter, referred to as "ERE") on chromosomal DNA.
(3) Stage III where the complex of the ER dimer and the ERE (i.e., ER/ERE complex) is activated to express the gene positioning downstream of the ERE.

Based on the idea mentioned above, the method has been devised for detecting an endocrine disrupting chemicals, as described later.

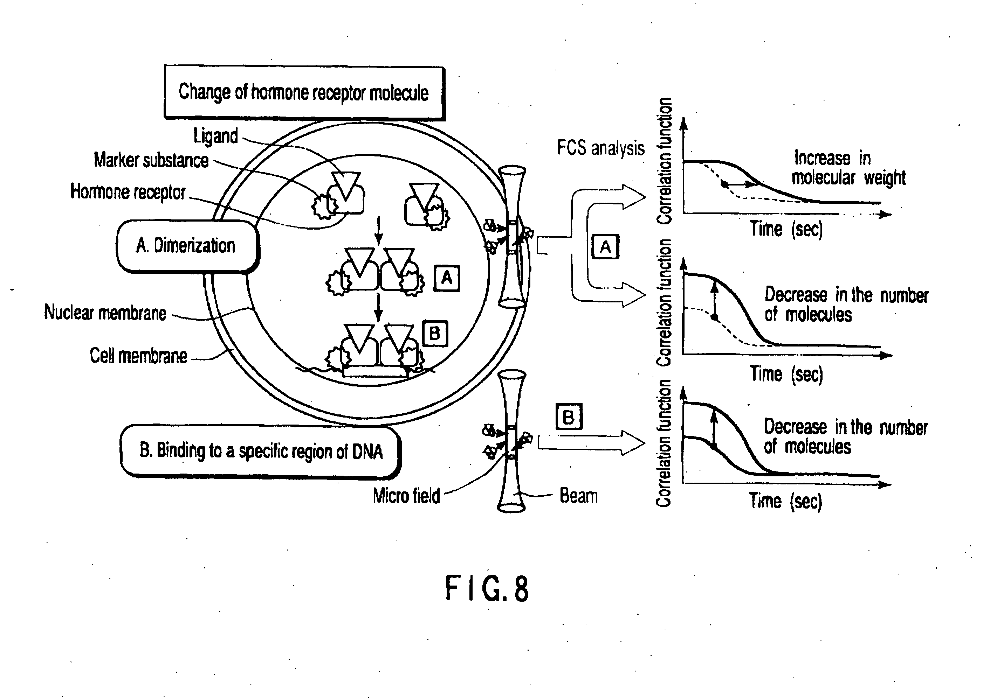

FIG. 8 shows a schematic view where the intracellular signal transduction of the hormone receptor molecule is detected by FCS. More specifically, "A" in FIG. 8 shows that the molecular weight increases and the number of the molecules decreases as a result of dimerization, and that these events are detected by FCS as a change of an autocorrelation function. "B" in FIG. 8 shows that the number of the molecules decreases by the binding of the molecules to the DNA specific region, and that this event is detected by FCS as a change of an autocorrelation function.

### (1) Detection in the stage I where ER is dimerized.

### (The case where FCS is used for detection)

According to a method of making the fusion protein mentioned above, a fusion gene comprising an ER gene and a green fluorescent protein (GFP) gene is prepared, and it is introduced into a cell, thereby allowing the expression of the ER/GFP fusion protein in the cell.
If ER binds a ligand (estrogen-like endocrine disrupting chemicals), thereby forming ER dimer, the formation of the dimer can be detected by FCS as a change in molecular weight and a change in number of molecules of the ER/GFP fusion fluorescent protein (see FIG, 8, A (dimerization)).

Further, in the FCS detection, the ER/GFP fusion protein expressed in a cell may be extracted from the cell, purified, and used.

### (The case where a change in wavelength of fluorescence is detected by fluorescence microscope)

It is known that ER has two subtypes, ERα and ERβ. Dimers such as αα, αβ, and ββ are formed.

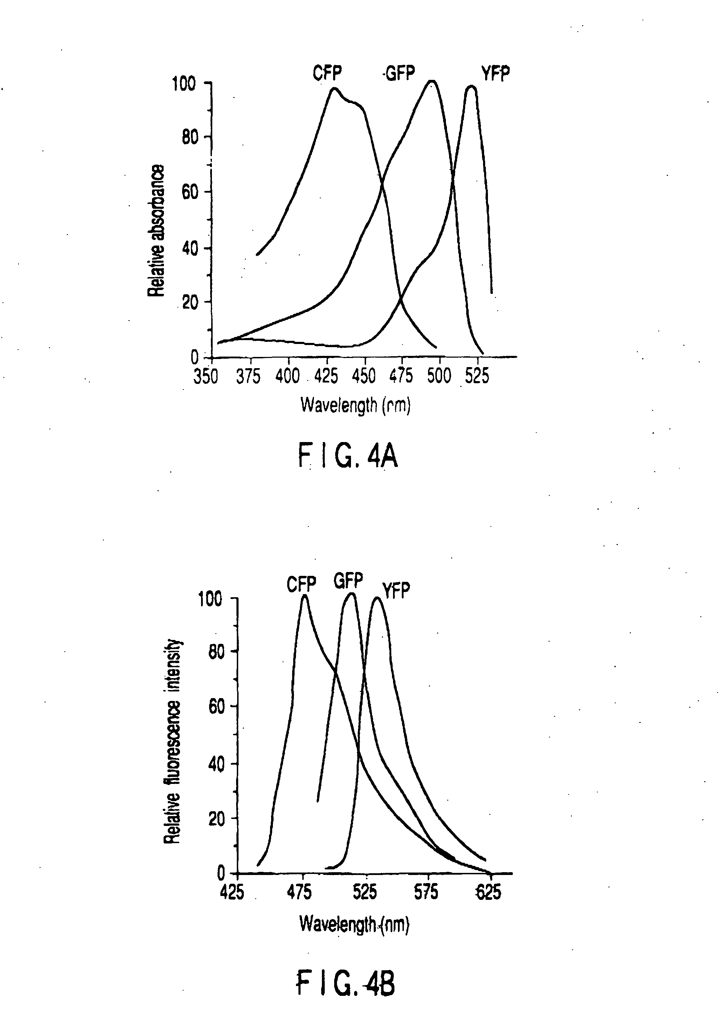

The fusion genes are prepared by connecting ERα and ERβ genes to genes coding fluorescent proteins CFP and YFP, respectively, thereby making ERα/CFP and ERβ/YEP fusion proteins, respectively. The CFP used herein is a cyan fluorescent protein (a maximum excitation wavelength: 433, 453 nm/maximum fluorescence emission wavelength 475, 501 nm). The YFP used herein is a yellow fluorescent protein (a maximum excitation wavelength: 513 nm/maximum fluorescence emission wavelength 527 nm). The characteristics of the excitation wavelengths and the fluorescence emission wavelengths of the fluorescent proteins CFP and YFP are shown in FIGS. 4A and 4B.

The ERα/CFP fusion protein emits fluorescence of 501 nm by irradiating light of 453 nm in wavelength, whereas ERβ/YEP protein is not excited by irradiation of light near 453 nm in wavelength. Accordingly, no fluorescence is emitted from ERβ/YEP. Therefore, in the case of no ligand, each of the fusion proteins is present independently, so that only the ERα/CFP is excited by irradiation of light near 453 nm, emitting blue fluorescence.

On the other hand, when the dimer of ERα/CFP and ERβ/YEP is formed in the presence of a ligand, fluorescence of 501 nm is first emitted from the ERα/CFP fusion protein by irradiating with light of 453 nm in wavelength. The fluorescence thus emitted then excites the ERβ/YEP fusion protein, which is present in close proximity to the ERα/CFP fusion protein because of the dimer formation. As a result, yellow fluorescence near 527 nm is emitted from the ERβ/YEP fusion protein. Therefore, the presence and absence of the dimer of ERα and ERβ can be monitored, by checking whether or not fluorescence near 527 nm is obtained by the irradiation of light near 453 nm. The dimerization of ERα and ERβ can be detected by a change in wavelength of fluorescence emitted.

Alternatively, if individual ERα receptors are labeled with CFP and YFP, respectively, it is also possible to detect an αα dimer. Similarly, if individual ERβ receptors are labeled with CFP and YFP, respectively, it is also possible to detect a ββ dimer.

### (2) Detection in stage II where the ER dimer binds to the estrogen responsive element sequence (ERE)

### (Detection system inside a cell)

The fusion gene comprising the ER gene and the GFP gene is prepared, and it is introduced into a cell, thereby allowing the expression of the ER/GFP fusion protein in the cell.

ER binds a ligand (estrogen-like endocrine disrupting chemicals), subsequently forming a dimer, and the dimer further binds to the ERE sequence of chromosomal DNA in a nucleus. This phenomenon can be detected by FCS based on a change in number of the fluorescent molecules of the ER/GFP fusion protein, more specifically, a decrease in number of the fluorescent molecules of the ER/GFP fusion protein suspended in the nucleus (see FIG. 8, B (binding to a specific region of DNA).

Alternatively, this phenomenon can be detected based on the change in intracellular distribution of the optical signal emitted from the fluorescent molecules. More specifically, the state of the binding of the intranuclear ER/GFP fusion fluorescent protein to the ERE sequence of the chromosomal DNA can be detected, by means of a fluorescence microscope, by comparing with the state where the fluorescent protein does not bind to the ERE sequence (the state where the fluorescent protein floats in the nucleus) based on the difference in the intranuclear distribution of fluorescence.

Further, when FCS is used for detection, the ER/GFP fusion protein expressed in a cell may be extracted from the cell, purified, and used, as described below.

### (Cell-free detection system)

The fusion gene comprising the ER gene and GFP gene is prepared and then the ER/GFP fusion protein is produced and purified in a large amount. On the other hand, 15 mer of oligonucleotide, ERE, is synthesized. Both preparations are suspended together in a solution, thereby constructing ER/ERE system without using cells. When a test substance is added to the ER/ERE system, the ER binds a ligand, forming a dimer, which further binds to ERE. The phenomenon can be detected by FCS, based on the structural change and the molecular-weight change of the fluorescent molecule of the ER/GFP fusion protein.

### (3) Detection in the stage III where a target gene is transcribed and translated

The ER/GFP fusion gene is transfected into a cell. In addition, a reporter gene, which is a gene capable of expressing an optical marker substance, is further introduced into the cell. In this way, an assay system, where the gene encoding the optical marker substance is transcribed, when ER binds a ligand, is constructed. In other words, the assay system is constructed by transfecting the reporter gene, which has an estrogen responsive element sequence in its expression regulatory region and is capable of expressing the optical marker substance, into the cultured cells. When a test substance is added, the amount of the product of the reporter gene, that is, the amount of the fluorescence of the optical marker substance, is detected by FCS, a fluorescence microscope, microplate reader, or the like. In this manner, an estrogen-like endocrine disrupting chemicals can be detected.

In addition, as the fluorescent protein to be used in the aforementioned detection system, another fluorescent protein such as CFP and YEP, besides GFP, may be used. Similarly, with regard to the fluorescent protein to be used as product of the reporter gene, any fluorescent protein may be used. Furthermore, another hormone receptor such as a glucocorticoid receptor, other than an estrogen receptor, may be used as the receptor molecule.

As explained in the above, in an attempt to detect the time-dependent intracellular movement of the hormone receptor molecule, the present inventors tried to label the intracellular hormone receptor molecule while the cell are maintained alive. To attain this, the present inventors employed a means for expressing the fusion gene comprising a fluorescent-substance gene and a hormone receptor gene, as described above.

On the other hand, the FCS analysis has been exclusively used to detect the fluctuation of the fluorescent molecules in a cell-free solution system. The cell-free solution system used herein dose not refer to an intracellular system expressing a fluorescence-labeled intracellular biological molecule, but refers to a solution system having fluorescence-labeled molecules suspended therein. In the case of the cell-free solution system, it has been relatively easy to adjust the number of the fluorescent molecules before FCS measurement. In the FCS measurement, the concentration of the fluorescent molecules in a sample must fall within the limited range to which a proper measurement can be performed. If the concentration in the sample is lower than or higher than the range, the proper measurement cannot be performed. Hence, it is necessary to perform the operation of adjusting the number of the fluorescent molecules.

However, in this invention, it is difficult to control the concentration (the amount expressed) of the fluorescence-labeled biological molecules in the cell, which is obtained by preparing a fusion gene of a target gene with a fluorescent protein gene and transfecting the fusion gene into the cell. As a matter of fact, it has a lot of difficulty to reproduce, in a cell, an optimal intracellular concentration of the fluorescent molecules for FCS measurement, every time the fluorescent-molecule-expressing cells are prepared by introducing the gene thereof. It is therefore important to establish a cell line where the fluorescence-labeled biological molecules are stably expressed at a suitable concentration for the FCS measurement, in order not only to omit unnecessary labors of using the cells impossible of the FCS measurement, but also to obtain data with a good reproducibility in analysis of physiological function. Furthermore, the construction of an experimental system using an established cell line suitable for the FCS measurement is very important in terms of establishing an evaluation system for biological function.

We found the aforementioned problem in the course of making the present invention, and studied to make a uniform cell system suitable for the FCS measurement with the view of detecting a substance interacting with a hormone receptor. Further, by using this established cell line, we worked for establishing an evaluation system analogous to a living body, which may substitute for test of an animal individual.

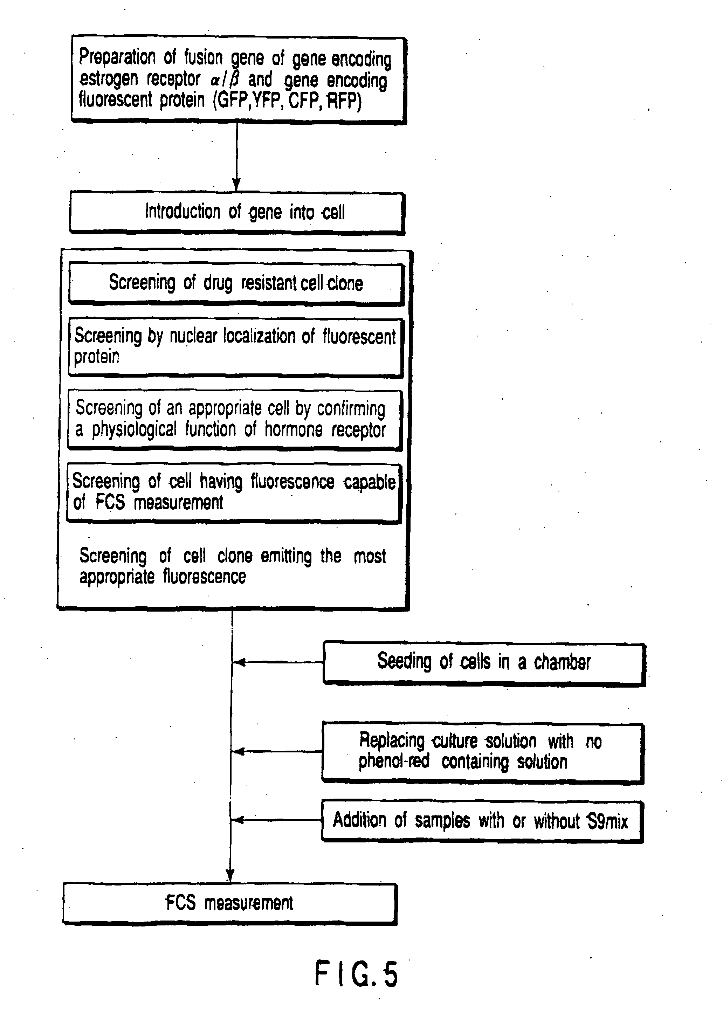

Hence, as a further preferable embodiment of the present invention, means A-D described below are employed. FIG. 5 shows a schematic flow-chart explaining a means for screening a cell clone suitable for FCS measurement.

### A. Screening of drug resistant cell clone

If the fusion gene comprising a hormone receptor gene and a marker substance gene is transiently expressed, it cannot be used as a secure experiment system. Therefore, a gene vector is designed in such a manner that the fusion gene has a drug resistant gene such as an antibiotic resistant gene (Geneticin, neomycin, kanamycin and the like). By the construction of such a gene vector, it becomes possible to screen a cell clone, which is constitutively expressing a fluorescent label of the intracellular biological molecule.

### B. Screening of the cells suitable for FCS measurement

In the present invention, the cell suitable for FCS measurement means the cell where the intensity of the detectable optical signal can be detected by FCS, and where the labeled hormone receptor therein maintains its physiological function and an evaluation system analogous to a living body is constructed.

### B-1. Screening of appropriate cells based on the intensity of an optical signal emitted from a cell

As described above, a hormone receptor protein is labeled with a fluorescent substance capable of emitting a signal and then placed in cells. Subsequently, from the cells, a cell emitting an optical signal having the most appropriate intensity is screened.

In FCS measurement, a confocal region is as small as 1 fL (femto liter). If the number of fluorescent molecules contained in the region dose not fall within the range from about 0.1 to 100, it is impossible to measure the molecular fluctuation suitably. More specifically, since the concentration of 10⁻⁷ to 10⁻⁹ M is the most suitable as concentration of the fluorescent molecule, it is necessary to screen the cells where fluorescence amount of fluorescent molecules is expressed optimally.

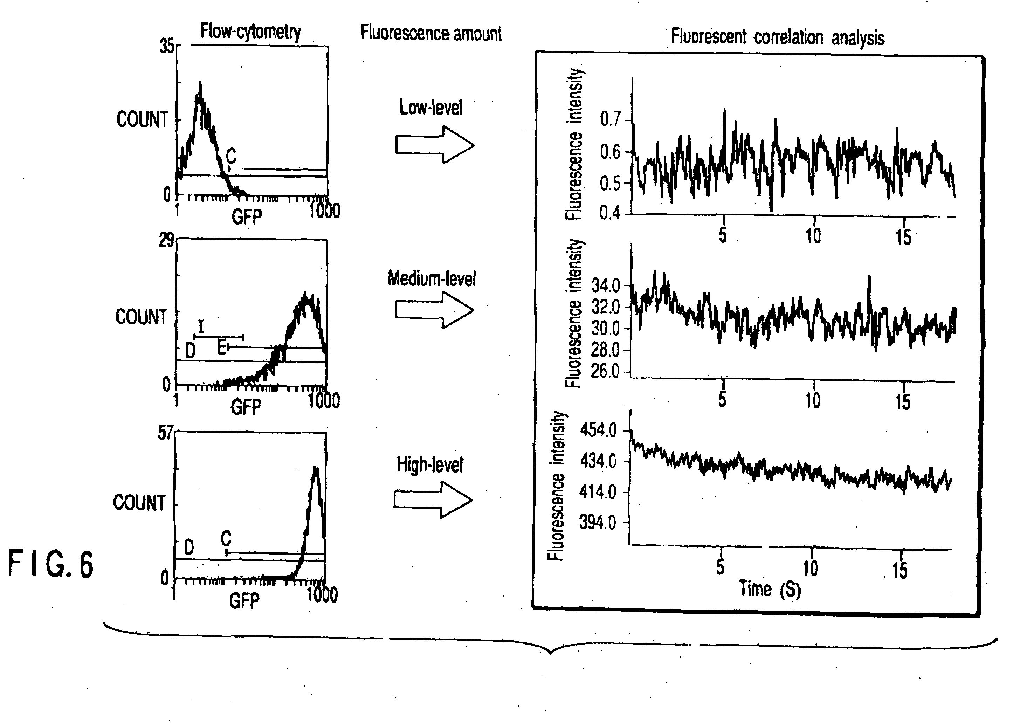

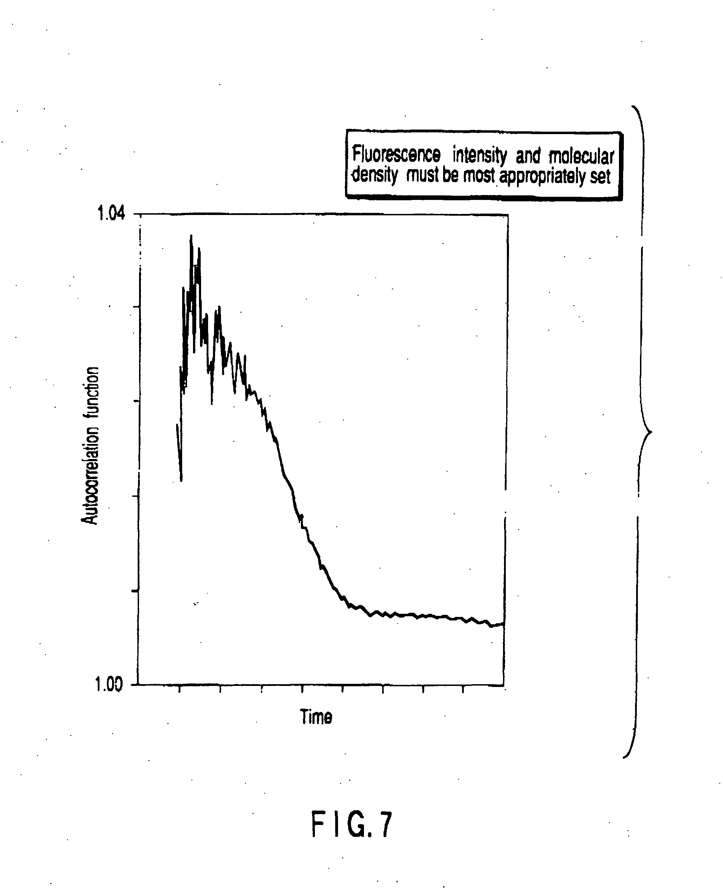

To measure the receptor molecules labeled with fluorescence by FCS, the concentration of the receptor molecules in a cell must fall within a measurable molecular-concentration range. If the concentration is larger or lower than the range, the measurement cannot be performed. This is explained in FIG. 6. FIG. 6 shows, sequentially from the above, the case where the number of fluorescent molecules is lower than the requisite concentration of the fluorescent molecules for FCS measurement, the case where the number of fluorescent molecules is within the appropriate range and moderate, and the case where the number of fluorescent molecules is larger than the appropriate range, respectively. In both cases where the number of fluorescent molecules is smaller and larger than the appropriate range, it is impossible to appropriately set the autocorrelation function exhibiting the movement of a target molecule. On the other hand, in the case where the number of the fluorescent molecules falls within the appropriate range, it is possible to appropriately set the autocorrelation function exhibiting the movement of the target molecule, as shown in FIG. 7.

Hence, in order to screen the cell emitting an optical signal appropriate for FCS measurement, it is necessary to determine the fluorescence qualitatively and quantitatively. Conceivable analytical methods for fluorescence will be shown below.

(a) A fluorescence microscope is generally used since cells can be observed while being cultured. However, the fluorescence intensity cannot be quantified. (b) Flow cytometry is excellent, since the fluorescence amount of each cell and an average fluorescence amount of the cells can be quantified as to a plurality of cells, and therefore the fluorescence intensity can be compared between cell clones. In the flow cytometry, the fluorescence of the cells can be measured while maintaining the cells alive. However, the flow cytometry has a disadvantage in that time and effort are required for suspending the cells. (c) Similarly, a fluorescence plate reader is excellent, since an average fluorescence amount of the cells can be quantified as to a plurality of cells, and therefore the fluorescence intensity can be compared between cell clones. Furthermore, if cells can be cultured on the fluorescent plate, it is possible to measure numerous cell clones at the same time.

In the present invention, the flow cytometry (FCM) is chosen from the aforementioned analytic methods. Using the flow cytometry, the fluorescence intensity per cell is quantified as to each clone. In this way, the fluorescence intensity per cell is quantitatively determined before FCS measurement, and this intensity value is checked with a result of analysis by the PCS measurement. Based on this check, it is possible to screen the cells having an optimal number of fluorescent molecules. By using this method, the present inventors found that the fluorescence intensity per cell suitable for the FCS measurement, which is determined by FCM, is 0.2-240, preferably 0.2-60, provided that the value of a negative control (no gene introduced cell) is 0.1.

Further, the means for quantifying the fluorescence per cell is not limited to FCM. Other methods such as a non-flow type of Laser scanning cytometer (LSC) may be used. It is preferable that the fluorescence intensity be appropriately defined depending upon the measurement device, types of fluorescent reagents, measurement conditions, measurement environment, and the like. For example, the fluorescence intensity may be expressed by the ratio of the fluorescence intensity, which can be determined based on value of the auto-fluorescence in a region (e.g., a cell) having no fusion gene introduced therein. When the ratio of the fluorescence intensity is employed, cells suitable for FCS measurement are cells whose ratio of the fluorescence intensity may fall within the range of 1-1200, preferably 1-300.

### B-2. Screening of proper cells based on a confirmation of physiological function of a fluorescence-labeled hormone receptor

It is necessary to confirm that the molecule expressed by the gene introduced into a cell has its physiological function. To attain this, it is preferable to confirm and screen the cell clones having the physiological function from numerous cell clones by an appropriate method.

In the present invention, the physiological function of the hormone receptor refers to both the function as a protein capable of emitting an optical signal and the function as the hormone receptor.

First, the function as the fluorescent protein can be certified by observing qualitatively and confirming emission of fluorescence by means of a fluorescence microscope.

Next, the function as the hormone receptor can be certified by observing the localization of fluorescence in a nucleus by a phase-contrast fluorescence microscope, since the hormone receptor is known to be localized in a nucleus from a physiological point of view. Thereby, it is also confirmed that the gene coding the hormone receptor holds a nuclear localization signal. Alternatively, the function as the hormone receptor may be confirmed by use of immunoassay (flow cytometry, Western blotting analysis) using an antibody to the hormone receptor.

### C. Removal of a substance inhibiting FCS measurement.

In the present invention, if a pH indicator (phenol red) is contained in a culture medium for the cells expressing a marker-labeled protein, the pH indicator has a hormone activity and also emits fluorescence. As a result, FCS measurement is inhibited by the non-specifically emitting fluorescence from the pH indicator. Furthermore, carbon dioxide gas has a physiological pH buffering action to the cell culture medium. However, the carbon dioxide gas cannot be supplied during the FCS measurement, so that the pH of the culture medium shifts to alkali, which has toxic effect on living cells.

To overcome the aforementioned problems, as the cell culture medium to be used in the FCS measurement, it is preferable that the culture medium is made of a stable composition containing no pH indicator (phenol red) and having a neutral buffering action. More specifically, since the conventional cell culturing conditions (CO₂ 5%, 37°C) cannot be employed during the FCS measurement, it is preferable to use a culture medium and whose physiological pH can be controlled by the buffering action which is not depending upon CO₂. Examples of such a culture medium include Leibovitz-15 medium, 10-20 mM HEPES (N-2-hydroxyethylpiperazine-N'-2-ethanesulfonic acid), etc..

### D. Pretreatment of a test substance with an extract from a living body, which has a drug metabolic activity

After the chemical substance to be investigated (hereinafter, referred to as "test substance") is actually taken into a living body such as a human body, it may affect the living body by the following two mechanisms. One is that the test substance directly affects the living body without being chemical structurally modified. The other one is that the test substance is metabolized by the enzyme system in the living body and the resultant metabolite acts upon the living body. As a matter of fact, when a chemical having an endocrine disrupting activity is taken into a human body, it first enters the liver. Therefore, it may be considered that the enzymatic metabolite of the test substance exhibits an endocrine disrupting activity. In the following, we will explain a method for detecting the endocrine disrupting activity of such enzymatic metabolite outside the living body.

A test substance is brought into contact with an extract from a living body (e.g., liver S9 fraction) having a drug metabolic activity, immediately before or at the same time the test substance is added to a test system. Thereby, after the test substance is chemically modified in the structure by metabolism, the endocrine disrupting activity is detected in accordance with the manner mentioned above. In parallel with the this detection, the control test may be also performed, where the test substance is directly added to the test system for the endocrine disrupting activity without being treated with the extract from the living body having a drug metabolic activity.

Consequently, it is possible to investigate both the direct effect of the test substance itself (without chemical modification) on the living body and the effect of the metabolite of the test substance (metabolized by the enzyme system and modified chemically) on the living body. Accordingly, whether or not the metabolite of the test substance has the endocrine disrupting activity can be also evaluated, although the endocrine disrupting activity of the metabolite cannot be detected by means of the conventional methods in which a test substance is directly added to the test system. Thereby, the endocrine disrupting activity of a chemical substance can be evaluated with more accuracy.

### Examples

Now, examples of the present invention will be explained.

### [Cloning of a human estrogen receptor β (hERβ) gene]

The following primers were designed for cloning the human estrogen receptor β (hERβ) gene on the basis of the nucleotide sequence of hERβ gene.

PCR was performed in the following conditions by using the primers and a human testis cDNA library (Clontech) as a template. More specifically, PCR was performed using the human testis cDNA library (about 400 ng) as a template and in a PCR reaction solution (50 *µ*L) containing the primers (4 µM for each), 0.2 mM dNTPs (dATP, dCTP, dGTP, and dTTP, 0.2 mM for each), 40 mM Tricine-KOH, 15 mM KOAC, 3.5 mM Mg(OAc)₂, 3.75 µg/mL BSA, 0.005% Tween-20, 0.005% Nonidet-P40 and Advantage 2 Polymerase Mix (Clontech #8430-1)(5 µL).

The PCR was performed as follows: After an initial denaturing step was performed at 95°C for 4 minutes, 7 cycles of 95°C for one minute, 62°C for one minute, and 72°C for 2.5 minutes, subsequently 25 cycles of 95°C for one minute, 58°C for one minute, and 72°C for 2.5 minutes were performed. After a final reaction was performed at 72°C for 10 minutes, the reaction product was stored at 4°C.

An aliquot (5 *µ*L) of the PCR product was subjected to 1% agarose gel electrophoresis. After it was confirmed that the molecular size of the product is almost the same as that of a target product, ERβ, the cloning of the PCR product was performed by using a TA cloning kit (Clontech #K1901-1). More specifically, the PCR product (2 µL) obtained within 24 hours after the PCR, was incubated at 14°C for 12 hours or more in a reaction solution (10 µL) containing 6 mM Tris-HCL, 6 mM MgCl₂, 5 mM NaCl, 0.1 mg/mL BSA, 7 mM β-mercaptoethanol, 0.1 mM ATP, 2 mM dithiothreitol, 1 mM spermidine, 50 ng of pT-Adv vector, and 4.0 Weiss units of T4 DNA ligase.

Thereafter, TOP10F' E. coli (Clontech #K1901-1) was transfected with using a ligation reaction solution (2 µL) in accordance with the protocol attached to the kit.

A plasmid was isolated from the resultant transfectant by use of NucleoBond Plasmid Kit (Clontech #K3001-1) and subjected to nucleotide sequencing. The nucleotide sequencing was performed by a PE Biosystems 377XL apparatus in accordance with a Dye Terminator Cycle Sequencing method.

As a result of the sequencing, an insert DNA sequence of the obtained clone was the same as that of 53-1735 bp of the human estrogen receptor β gene.

This sequence includes full length of the sequence from the initiation codon to the termination codon, which is the coding region of the human estrogen receptor β protein.

### [Preparation of a fusion gene and construction of an expression plasmid]

The hERβ gene thus isolated was subcloned into pEGFP-C1 vector (GenBank Accession #; U55763), thereby preparing a fusion gene comprising the hERβ gene and a GFP (green fluorescent protein) gene derived from the jellyfish Aequorea victoria, and constructing a plasmid vector for expressing in mammalian cultured cells. The schematic view of a pEGFP-C1 vector is shown in FIG. 1.

Plasmid hERβ/pT-Adv (about 20 µg) prepared as described above was digested with two types of restriction enzymes, HindIII and BamHI. Thereafter, the total amount of the reaction mixture was subjected to electrophoresis using 1.0% low-melting point agarose gel. After the gel was stained with ethidium bromide, a band portion corresponding to the ERβ gene fragment of about 1.7 kb was cleaved out from the gel, while visually observing the size of the DNA fragment on a UV transilluminator. From the gel cleaved out, the ERβ fragment was purified by using Concert Rapid Gel Extraction System (GIBCO BRL 11456-019).

Similarly, a pEGFP-C1 vector (about 20 µg) was digested with two types of restriction enzymes, HindIII and BamHI, making the vector linear. After the total amount of the reaction mixture was subjected to electrophoresis using 1.0% low-melting point agarose gel, a band portion corresponding to the linear pEGFP-C1 fragment of about 4.7 kb was cleaved out. From the gel cleaved out, a pEGFP-C1 vector was purified by using a Concert Rapid Gel Extraction System (GIBCO BRL 11456-019).

A DNA solution (about 10 µL) containing the vector fragment (0.03 pmol) and the hERβ fragment (0.1 pmol) purified as mentioned above were mixed with the equal amount of solution I of TAKARA DNA Ligation System Ver. 2 (TAKARA #6022). The mixture was incubated at 16°C for about 12 hours, thereby performing ligation.

E. coli DH5α (TAKARA #9057) was transformed with the resultant reaction mixture (10 *µ*L) thus obtained. A plasmid was isolated from the transformants obtained by NucleoBond Plasmid Kit (Clontech #k3001-1) and subjected to nucleotide sequencing. The nucluotide sequencing was performed by PE Biosystems 377XL apparatus in accordance with a Dye Terminator Cycle

### Sequencing method.

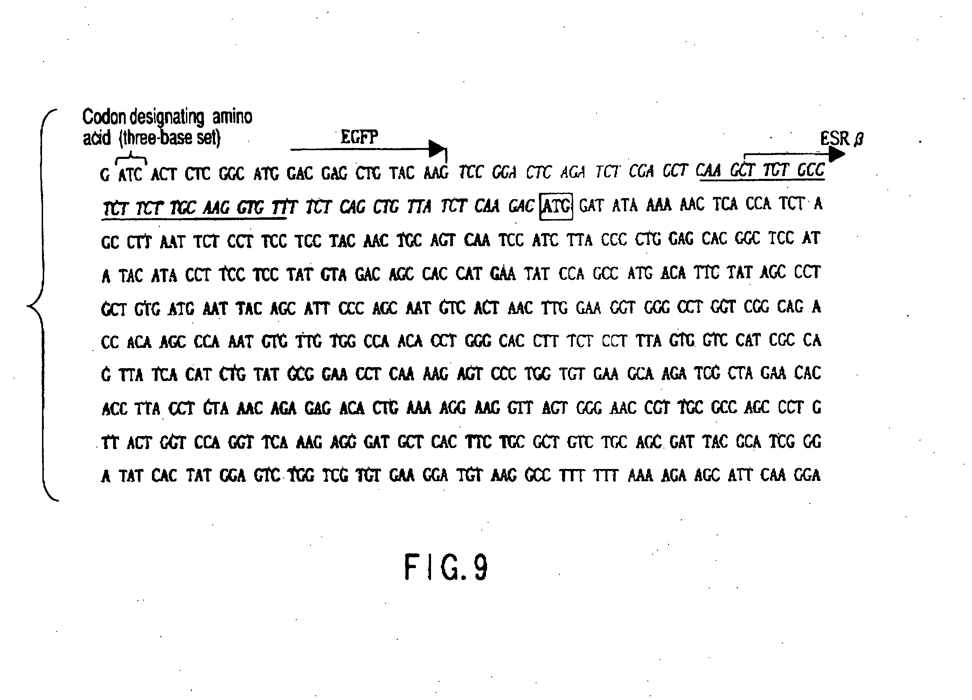

A part of the nucleotide sequence of the fusion gene is shown in FIG. 9. FIG. 9 shows that the gene coding the green fluorescent protein (EGFP in the figure), the multi-cloning site, and the hERβ gene (hERβ in the figure) are arranged sequentially in the direction from 5 to 3'. The sequence (GT GCC TCT TCT TGC AAG GTG TT) underlined in the figure indicates the portion of a primer sequence used in the cloning of hERβ gene. The sequence (AA GCT T) underscored with a wavy line in the figure indicates a restriction site of HindIII. The ATG site enclosed with a square frame in the figure indicates a transcription initiation codon of the hERβ gene.

### [Transfection of a human cultured cell strain with an expression plasmid]

A cultured cell strain MDA-MB-231, which is a cultured cell line derived from human mammary cancer tissue, was transfected with the fusion-gene-expression plasmid GERb3-9-1 constructed as described above. The cultured cell strain MDA-MB-231 derived from human mammary cancer tissue was purchased from ATCC and cultured in L-15 medium containing 10% FBS (Fetal Bovine serum) at 37°C in humidified incubator. The MDA-MB-231 cells were treated with trypsin to disperse them, and then about 2 × 105 of the MDA-MB-231 cells were seeded on a 60 mm petri dish, one day before the transfection. The plasmid GERb3-9-1 was introduced into the cells by means of lipofectin (GIBCO #10964-013), on the following day.

A DNA solution containing the plasmid GERb3-9-1 (2 µg) was diluted with 250 µL of serum-free L-15 medium. To this solution, a plus reagent (8 µL) was added and allowed to stand at room temperature for 15 minutes. Thereafter, a solution containing a lipofectamine reagent (12 µL) diluted with the serum-free L-15 medium (250 µL), was added to the solution mixture of the DNA/plus reagent, and allowed to stand at room temperature for a further 15 minutes. During this period, the culture solution was removed from the petri dish having the cells seeded on the previous day, and 2 mL of the serum-free L-15 medium was added in place of the culture solution. After 15 minutes, 2.5 mL of the solution mixture of the DNA/plus reagent/lipofectamine previously prepared was added to the petri dish whose culture solution was previously replaced with the serum-free L-15 medium (2 mL). The petri dish was incubated for 3 hours at 37°C in a humidified incubator. After 3 hours, the culture solution was replaced with 5 mL of general L-15 medium containing 10% FBS, and incubated for a further 24 hours or more. In this manner, the MDA-MB-231 cultured cell strain was transfected with the plasmid GERb3-9-1.

### [Construction of stable transfected cultured cell line and expression of the fusion protein in the cell line]

The cultured cell thus transfected was dispersed with the treatment of trypsin, diluted and seeded on a new petri dish. On the following day, G418 antibiotic was added so as to become a final concentration of 600 µg/mL, for selection of transfectants.
Thereafter, incubation was continued while a culture solution (selective culture solution, L-15 medium containing 10% FBS and 600 µg/mL of G418) was replaced every 3-4 days. In this manner, a stable transfectant was screened with G418. About two weeks later, a stable cell clone acquiring G418 resistance was isolated. In this manner, a stable tranfected cultured cell line with the plasmid GERb3-9-1 was established. It was difficult to obtain the cell clone stably expressing the fusion protein of an estrogen receptor and GFP, herein. This is because the cell expressing the fusion protein at a relatively a high level died several days after the trasfection. In the present invention, the transfection was performed a plurality of times, and a large amount of cells was subjected to screening. As a result, it was possible to obtain cell line (clone) stably expressing the fusion protein.

FIG. 2 shows a photomicrograph, by a fluorescence microscope, of the transfected cultured cell line thus established. It was observed that the GFP/hERβ fusion protein encoded by the plasmid GERb3-9-1 was expressed in a cell and localized in the nucleus of the cell.

### [Labeling of intracellular estrogen receptor molecule with a fluorescence antibody]

### Seeding of Cells

1. Human mammary cancer cultured cells MCF-7 in a logarithmic growth phase were released with trypsin + EDTA (0.25% trypsin-EDTA: GIBCO CAT #25200-056) and washed (centrifuged at 300 × g for 5 minutes) twice with D-PBS (phosphate buffer).
2. The cells were divided to 10 test tubes for each antibody, so as to become a concentration of about 5 × 10⁵ cells per tube, using 50 *µ*L of D-PBS as dilution solution.

### Hypotonic Treatment for Cells

As a hypotonic solution for cells, an IntraPrep permeabilization Reagent corresponding to 50 tests (IMMUNOTECH (COULTER), CAT #2388) was used.
3. Intraprep reagent 1 (100 *µ*L) was added into the test tubes, and mixed well by voltex.
4. The mixture was incubated for 15 minutes at room temperature (18-25°C).
5. D-PBS (4 ml) was added to the mixture, and the resultant mixture was centrifuged at 300 × g for 5 min. at room temperature. The supernatant was removed by aspiration.
6. The cells were made loose by tapping. Then, IntraPrep Reagent 2 (100 µL) was added thereto, and the mixture was mixed gently without using voltex.
7. The mixture was incubated at room temperature for 15 minutes.
8. The resultant mixture was tapped gently with fingers for 1-2 seconds (without using Voltex).

### Addition of Primary Antibody

Anti-estrogen receptor monoclonal antibody (Mouse) (Affinity Bioreagents Inc., CAT #MA1-310) was used as an anti-ERα antibody, and an anti-estrogen receptor polyclonal antibody (Rabbit) (Affinity Bioreagents Inc., CAT #PA1. 1-313) was used as an anti-ERβ antibody.
9. The anti-ERα antibody or the anti-ERβ antibody was diluted with a dilution solution D-PBS to 200 folds. The resultant solution (50 µL) was added to a predetermined test tube and gently mixed.
10. The resultant mixture was incubated at room temperature in a dark place for 15 minutes.
11. D-PBS (4 mL) was added to the resultant mixture, and the resultant mixture was centrifuged (300 × g, 5 min) at room temperature. The supernatant was removed by aspiration. This washing was performed twice in total in the same manner as mentioned above.

### Addition of Secondary Antibody

0.5 mL of Molecular Probes, CAT #A-11029, Alexa 488/goat anti-mouse IgG (H+L) was used as the fluorescence-labeled anti-mouse antibody. 0.5 mL of Molecular Probes CAT #A-11037, Alexa 594/goat anti-rabbit IgG was used as the fluorescence-labeled anti-rabbit antibody.
12. The fluorescence-labeled anti-mouse antibody (Alexa 488) and the fluorescence-labeled anti-rabbit antibody (Alexa 594) were diluted with a dilution solution D-PBS to 100 folds and 200 folds, respectively. The solutions thus diluted were added to predetermined test tubes in an amount of 50 *µ*L and gently mixed.
13. Washing with D-PBS was performed three times.
14. The cells thus obtained were suspended in 500 µL of D-PBS containing 0.5% formaldehyde, and analyzed by a flow cytometer.

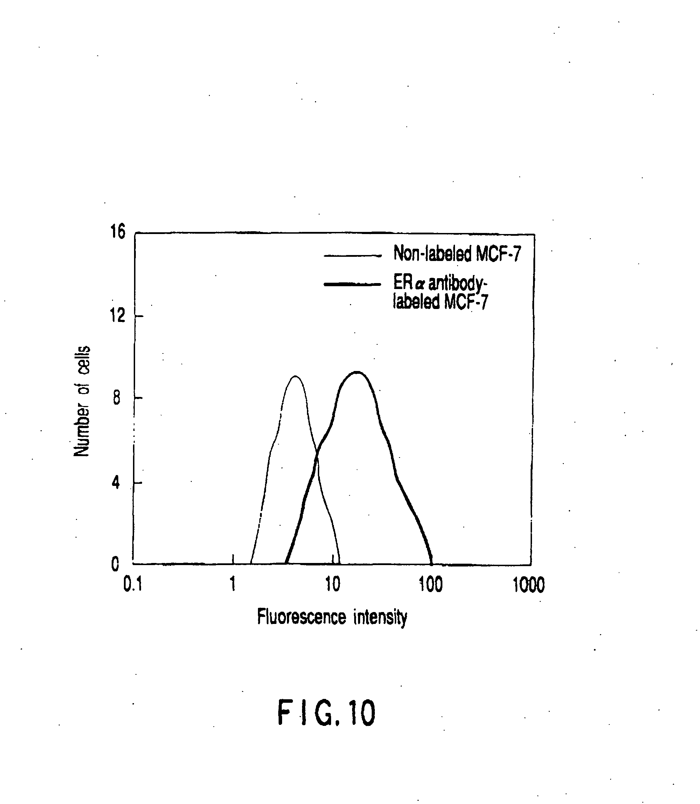

By the aforementioned method, an intracellular estrogen receptor was labeled with fluorescence. The analysis by flow cytometry shows that human mammary cancer cell MCF-7 expresses the estrogen receptor (ERα). The analytical result is shown in FIG. 10. The human mammary cancer cell MCF-7 did not express the estrogen receptor β (ERβ). On the other hand, cells derived from human mammary cancer tissue, MDA-MB-231, expressed neither ERα nor ERβ.

### [Comparison between the fluorescence intensity and FCS measurement data, concerning the cell having only a gene coding a fluorescent protein introduced therein]

MCF-7 cells were seeded on 60 mm petri dish at about 80% of confluence. The MCF-7 cells were transfected with an expression vectors pEGFP-c1, pEYFP-c1, pECFP-c1 (1-10 µg for each) containing a fluorescent-protein encoding gene, in accordance with a lipofection method by CLONfection (CLONTECH). Geneticin (600-800 µg/ml) was added to a growth medium for MCF-7 cells (MEM + 10% fetal bovine serum + nonessential amino acid + 1 mM sodium pyruvate). In this manner, a selective medium was prepared.
The cells obtained as described above were incubated for 2-3 weeks in a CO₂ incubator, while the medium was replaced with a fresh medium every 3-4 days. As a result, a selected clone was obtained.

The cell clones were observed by a fluorescence microscope using an optimal fluorescence filter. In the cells expressing the fluorescent proteins GFP and YFP, the emission of the fluorescence was observed, whereas a major fluorescence signal emitted from fluorescent protein CFP was not detected.

Further, the fluorescence intensity of the cell clones obtained was compared by flow cytometry (BECKMAN-COULTER, XL) using filters PF1 and PF2.

The method of analysis by flow cytometry will be explained below.
1. MCF-7 cell clones having only a fluorescent-protein-coding gene transfected therein, are cultured individually, and a cells of about 10⁶ are released with trypsin + EDTA and washed twice with D-PBS.
2. The resultant cells are placed in a tube only for flow cytometry, and subjected to flow cytometry.
3. Using the cell clones, gating of size and density of the cell is performed.
4. Fluorescence is appropriately corrected, if necessary, so as to make the fluorescence intensity of the resultant cell population optimal.

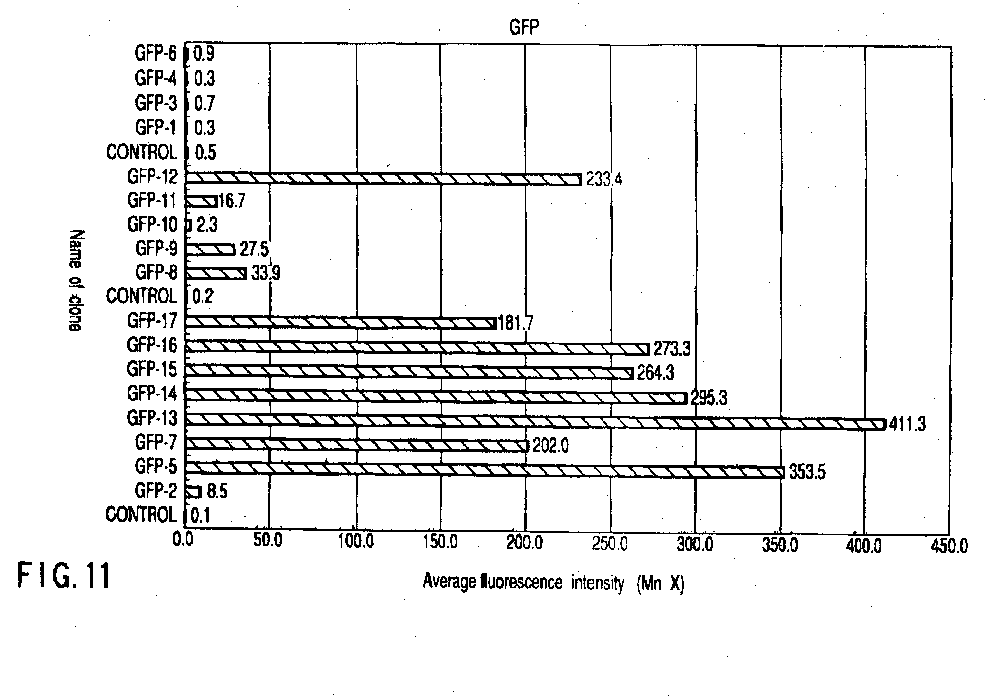

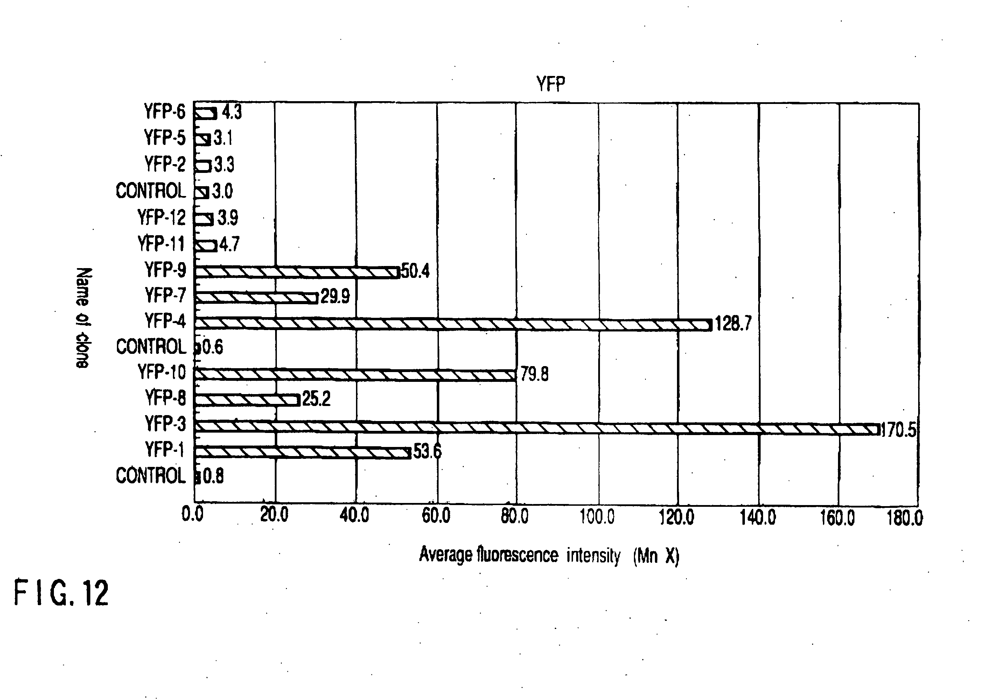

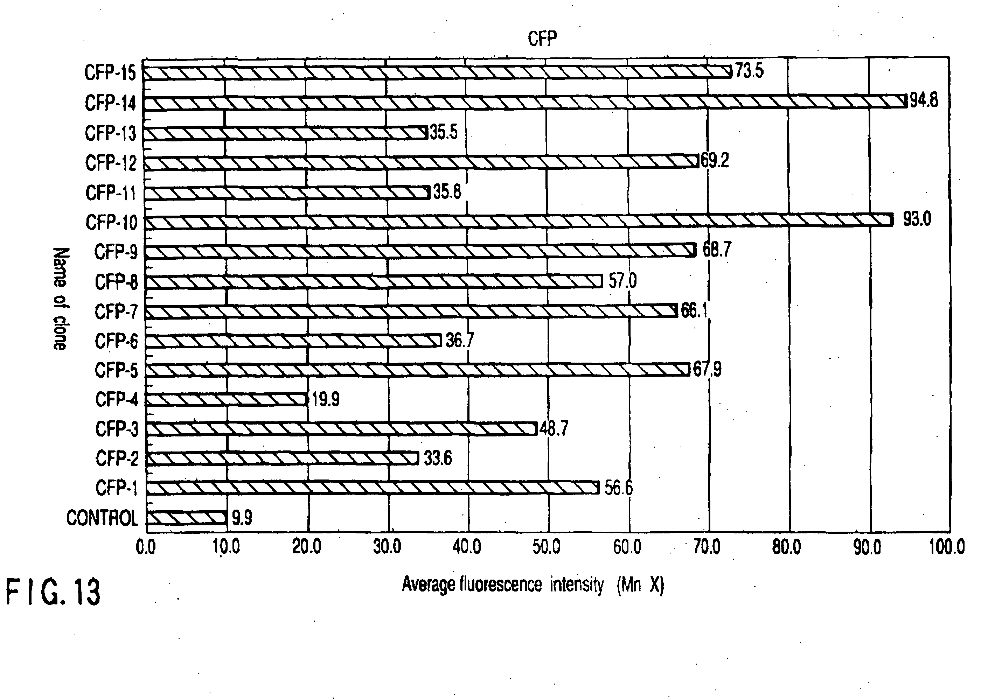

The measurement results of fluorescence intensity are shown in FIGS. 11-13.

Of them, in the cell clones GFP-5 and YFP-3 having different fluorescence intensity, FCS measurement data are compared. The FCS measurement is performed by ZEISS-made FCS (ConfoCor®) (Light source: Ar laser, Objective lens: 40 folds, Confocal region: 0.25 µm (radius) × 1.7 µm (height)). The fluorescence intensity of GFP-5 is high (average fluorescence intensity: 353.5 MnX), which exceeds an appropriate concentration of fluorescent molecules. YFP-3 has an appropriate concentration of the fluorescent molecule (average fluorescence intensity: 170.5 MnX), so that the fluorescence can be measured by FCS. The autocorrelation function of YFP-3 is shown in FIG. 7. With respect to the clone cells emitting no fluorescence, the autocorrelation function is not appropriately set.

In the case of GFP-5 exhibiting high fluorescence intensity, the number of the fluorescent molecules in a measurement region exceeds a limit of 100. For this reason, it is found that FCS measurement cannot be performed. On the other hand, FCS measurement is performed by adjusting the concentration of rohdamin 123 in a solution at 0.1-1000 nmol/L. Compared with the results of the FCS measurement using rohdamin 123, the concentration of the fluorescent molecules GFP-5 is assumed to be about 700 nM or more. From this, it is found that the most suitable concentration of the intracellular fluorescence-labeled molecules for the FCS measurement is 0.1-700 nmol/L. From this value, as a value of fluorescence intensity per cell which is measured by flow cytometry, a range of 0.2-240, preferably 0.2-60 is found to be most suitable for subjecting to the FCS measurement, provided that the intensity of a negative control (a no-gene-transfected cell) is set at 0.1. Therefore, the cell clone whose fluorescence intensity falls within the aforementioned range may be chosen for the FCS measurement. The means for quantifying fluorescence per each cell is not limited to FCM. Other methods such as a non-flow type of laser scanning cytometer (LSC) may be used. It is preferable that the fluorescence intensity be appropriately defined depending upon the measurement device, types of fluorescent reagents, measurement conditions, measurement environment, and the like. For example, the fluorescence intensity may be expressed by the ratio of the fluorescence intensity, which can be determined based on value of the auto-fluorescence in a region (e.g., a cell) having no fusion gene introduced therein. When the ratio of the fluorescence intensity is employed, cells suitable for FCS measurement are cells whose ratio of the fluorescence intensity may fall within the range of 1-1200, preferably 1-300.

As described above, it is possible to quantify the fluorescence intensity by using the flow cytometry. In this way, the fluorescence intensity of each cell clone can be numerically expressed. Therefore, a method of screening a cell clone for use of FCS analysis, which has an optimal density of fluorescent molecules, was established.

### [Detection of dimerization of the fusion protein followed by ligand binding]

The dimerization of the GEP/hERβ fusion protein, which is caused by binding of 17β estradiol, was detected by using the cultured cell clone #1 expressing the GFP/hERβ fusion gene previously established. The cultured cell clone #1 expressing the GFP/hERβ fusion gene was seeded in a Lab-Tek chamber (Nunc #136439) having 8 wells, and incubated in a humidified incubator at 37°C, overnight. Thereafter, the culture solution was removed, the cells were washed with PBS, and then the culture solution was replaced with serum-free and phenol-red-free L-15 medium. To this, 17β estradiol was added so as to be a final concentration of 10⁻⁷M. Thereafter, at predetermined intervals over 2 hours, the intranuclear behavior of the GFP/hERβ fusion protein was measured by ZEISS-made FCS (ConfoCor®).

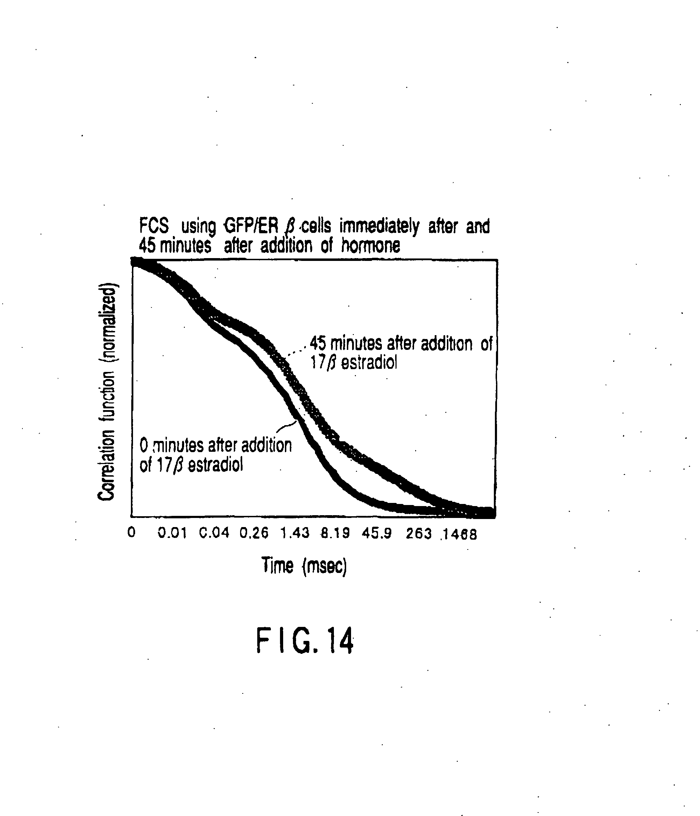

FIG. 14 shows FCS measurement data immediately after (0 minute) and 45 minutes after the addition of 17β estradiol. As is apparent from the measurement results, the diffusion time of the GFP/hERβ fusion protein increases at 45 minutes after the addition of 17β estradiol. It is determined that such measurement results were associated with the dimerization of GFP/hERβ fusion protein followed by binding of 17β estradiol. Such a reaction was not observed in the control cell expressing only GFP.

As described above, for the first time by the present invention, it was possible to detect the stage of the dimerization of estrogen receptor molecules.

### Effect of the invention

As previously explained, for the first time by the present invention, a new assay system for testing a substance interacting with an hormone receptor is established by use of the cell expressing the fluorescence/receptor fusion protein therein.

To construct the assay system of the present invention, a novel fusion gene is prepared a new fusion gene comprising a gene coding a hormone receptor protein and a gene coding a marker substance capable of emitting an optical signal, and constructed a recombinant vector containing the fusion gene therein. Subsequently, a transfected cell was created by introducing the recombinant vector. By virtue of this method, it is possible to express the fusion protein of the hormone receptor/marker substance in the transfected cell. As a result, it is possible to express the hormone receptor protein capable of emitting an optical signal in a living cell.

If a test substance is added to the cell expressing the hormone receptor protein capable of emitting an optical signal, it is possible to trace the behavior of the hormone receptor molecules (i.e., signal transduction) by using the optical signal. As a result, the endocrine disrupting activity of the test substance can be evaluated in a living cell.

If the fusion protein of the hormone receptor/fluorescent protein is constructed in a cell as described above, it is possible to directly analyze the binding of the test substance to the hormone receptor, while maintaining physiological function of the receptor. Furthermore, the time-dependent-behavior of the hormone receptor can be detected in a living cell. Therefore, this evaluation system is advantageous, since it is analogous to the system of a living body.

In addition, use of the cell expressing the fusion protein is advantageous in the following points. First, only a few numbers of cells are required, and a single cell is good enough to be measured.
Furthermore, it is possible to observe that intracellular localization of a target protein is changed by external stimulation.

On the other hand, it is also possible to evaluate the endocrine disrupting activity of a test substance by adding the test substance to the purified fusion protein.

The advantage of using the purified fusion protein resides in that a detection system can be established without contaminants as is present in the cell. As a result, the measurement can be performed with a reduced noise level. The purified fusion protein can be stored by freezing for a long time. Labor and time of maintaining cell by subculture are not required. Furthermore, it is easy to control the concentration of the target protein, reaction temperature and the like, so that experimental conditions can be optimized easily.

In the course of making the present invention, a system imitating a living-body was established by introducing and expressing the fusion protein of hormone receptor/fluorescent protein into a living cell. As a result, it became necessary to screen the cells exhibiting an expression amount of fluorescence suitable for FSC measurement. Hence, the expression amount of the fluorescent protein was quantified by the flow cytometry, and thereby the fluorescence intensity of the cell, which exhibits an expression amount of fluorescence suitable for FCS measurement, was expressed numerically. In this way, a simple method for screening a cell was is established. This method led to an establishment of a stable experimental system, where it is possible to detect the behavior of the fluorescent receptor molecules in a living cell with a good reproducibility and a high sensitivity. By the establishment of the stable experimental system, it further became possible to detect the dimerization of the hormone receptor in a living cell, although it could not be done by conventional method.

Furthermore, in the assay system of the present invention, various effects can be obtained as set forth below.

According to the method of the present invention, it is possible to detect at an early stage (at Stage I shown in FIG. 3) of intracellular signal transduction via the fluorescence/receptor fusion protein, which is induced by a chemical substance. It is therefore possible to detect the endocrine disrupting activity of the chemical substance within a few hours earlier than that of a conventional method.

According to the method of the present invention, it is possible to know up to which stage (stages I to III shown in FIG. 3) of the intracellular signal transduction a chemical substance induces via the receptor protein. Based on this, it is possible to evaluate whether the test substance works as an agonist or an antagonist.

Since the fusion protein comprising a fluorescent protein (e.g., GFP) and a receptor protein is prepared by means of gene engineering, and the behavior of the resultant fluorescent fusion protein is used as a detection indicator, it is possible to detect individual stages of the intracellular signal transduction, without adding a specific reagent as is used in a conventional method.

Individual stages of the intracellular signal transduction can be basically detected by using a single cell. Therefore, numerous cells are not required as is in the prior art. Furthermore, by means of using FCS for the detection of the intracellular signal transduction, it is also possible to detect the change in the behavior of a single molecule of the fluorescence/receptor fusion protein. As a result, the endocrine disrupting activity of a chemical substance present in an extremely small amount can be detected with a high sensitivity.

Since the method of the present invention using FCS measurement requires only a small amount of sample, it can be applied to an apparatus for High Through Put (HTP) method, which is operated at a high-speed and enables measurement of a small amount of sample.

Furthermore, by detecting a substance capable of binding to an estrogen receptor using the method of the present invention, a naturally-occurring female hormone present in a living body (in blood, urine) other than the endocrine disrupting chemicals can be detected. The method of the present invention has a high sensitivity to estrogen, so that it can detect an estrogen level varying depending upon a menstrual cycle and a high level of estrogen during the ovulation period. Due to the high sensitivity, the method of the present invention may be used in diagnosis for pregnancy.

### Industrial Applicability

As explained in the foregoing, the present invention is effective in testing a substance interacting with a hormone receptor, such as an endocrine disrupting chemicals.

## Claims

1. An in vitro method for testing a substance interacting with a hormone receptor, **characterized by** comprising:
(a) maintaining a hormone receptor protein labeled with a marker substance capable of emitting an optical signal and a test substance, under predeterminded conditions where a natural ligand is allowed to be bound to the receptor protein labeled with the marker substance and where, after binding the natural ligand, the hormone receptor protein is allowed to form a dimer of a complex of the hormone receptor protein/ligand, thereby inducing a change in a detectable characteristic of the optical signal emitted from the marker substance labeling the hormone receptor protein;
(b) detecting the optical signal emitted from the marker substance under the predetermined conditions; and
(c) comparing the characteristic of the optical signal detected in the step (b) with that of the optical signal detected from the marker substance labeling the hormone receptor protein in the absence of the test substance, thereby determining whether or not the test substance is a substance capable of interacting with the hormone receptor protein on the basis of the difference in both characteristics detected,
wherein the difference results from dimerization of the complex of the hormone receptor protein/test substance and the monomer.

2. An in vitro method for testing a substance interacting with a hormone receptor which comprises:
(a) maintaining a hormone receptor protein labeled with a marker substance capable of emitting an optical signal and a test substance, under predetermined conditions where a natural ligand is allowed to be bound to the receptor protein labeled with the marker substance and where, after binding the natural ligand, the hormone receptor protein is allowed to form a dimer of a complex of the hormone receptor protein/ligand and then the complex of the hormone receptor protein/ligand is allowed to be bound to a specific nucleotide sequence, thereby inducing a change in a detectable characteristic of the optical signal emitted from the marker substance labeling the hormone receptor protein;
(b) detecting the optical signal emitted from the marker substance under the predetermined conditions; and
(c) comparing the characteristic of the optical signal detected in the step (b) with that of the optical signal detected from the marker substance labeling the hormone receptor protein in the absence of the test substance, thereby determining whether or not the test substance is a substance capable of interacting with the hormone receptor protein on the basis of the difference in both characteristics detected,
wherein the difference results from the binding of the dimer of the complex of the hormone receptor protein/test substance to the specific nucleotide sequence.

3. A method according to claim 1 or 2, **characterized in that** the hormone receptor protein labeled with the marker substance capable of emitting an optical signal is a fusion protein comprising a hormone receptor protein and the marker substance capable of emitting an optical signal.

4. A method according to claim 1, **characterized in that** the change in a detectable characteristic of an optical signal is a change in wavelength of the optical signal.

5. A method according to claim 2, **characterized in that** the change in a detectable characteristic of an optical signal is a change in intracellular distribution of the optical signal.

6. A method according to any one of claims 1 to 3 and 5, **characterized in that** the change in a detectable characteristic of an optical signal is detected by fluorescence correlation spectroscopy (FCS).

7. A method according to any one of claims 1 to 3, **characterized by** comprising a step of bringing a test substance into contact with a cell, which is in a state of expressing therein the hormone receptor protein labeled with the marker substance capable of emitting an optical signal.

8. A method according to claim 7, **characterized in that** the step of detecting comprises the step of detecting an optical signal emitted from the marker substance in a cell.

9. A method according to claim 8, **characterized in that**, in the step of detecting, the optical signal is detected by focusing in an inner region of a cell.

10. A method according to any one of claims 1 to 3, **characterized by** comprising a step of mixing and reacting the test substance with the hormone receptor protein, which is labeled with the marker substance capable of emitting the optical signal, in a liquid where the protein extracted from a cell expressing itself is suspended.

11. A method according to claim 7, **characterized by** further comprising a step of screening a cell suitable for FCS detection from cells expressing the hormone receptor protein labeled with the marker substance capable of emitting an optical signal.

12. A method according to claim 11, **characterized by** comprising a step of screening a cell capable of FCS detection from cells expressing the hormone receptor protein labeled with the marker substance capable of emitting an optical signal, based on intensity of an optical signal emitted from the fluorescent substance.

13. A method according to claim 11, **characterized by** comprising a step of screening a cell whose intensity of an optical signal per cell falls within a range of 0.2 - 240, provided that intensity of a negative control (a cell containing no optical signal) is set at 0.1, from cells expressing the hormone receptor protein labeled with the marker substance capable of emitting an optical signal.

14. A method according to any one of claims 11 to 13, **characterized by** comprising a step of screening a cell where the labeled hormone receptor protein maintains its physiological function, from cells expressing the hormone receptor protein labeled with the marker substance capable of emitting an optical signal.

15. A method according to any one of claims 11 to 14, **characterized in that** a substance inhibiting FSC measurement is removed from a cell culture solution.

16. A method according to any one of claims 1 to 15, **characterized by** comprising a step of bringing the test substance into contact with an extract which has been obtained from a living body and has a drug metabolic activity.

17. A method according to any one of claims 1 to 16, wherein the hormone receptor protein is an estrogen receptor protein.

18. A method according to any one of claims 1 to 17, wherein the hormone receptor protein is human estrogen receptor β.

19. A method according to any one of claims 1 to 18, wherein the marker substance is a fluorescent protein.

## Patentansprüche

1. In vitro Verfahren zur Untersuchung einer Substanz, die mit einem Hormonrezeptor interagiert, **dadurch gekennzeichnet, dass** es umfasst:
a) Behalten eines Hormonrezeptorproteins, das mit einer Markersubstanz markiert ist, die in der Lage ist, ein optisches Signal zu emittieren sowie eine Testsubstanz, unter vorbestimmten Bedingungen, in denen ein natürlicher Ligand mit dem Rezeptorprotein, das mit der Markersubstanz markiert ist, binden kann, und in denen nach der Bindung des natürlichen Liganden, das Hormonrezeptorprotein ein Dimer eines Komplexes des Hormonrezeptorprotein/Ligand bilden kann, wobei eine Veränderung in einem detektierbaren Merkmal des optischen Signals, das von der Markersubstanz, die das Hormonrezeptorprotein markiert, induziert wird;
b) Detektieren des optischen Signals, das von der Markersubstanz unter den vorbestimmten Bedingungen emittiert wird; und
c) Vergleichen der Merkmale des optischen Signals, das in Schritt b) detektiert wurde, mit denjenigen des optischen Signals, das von der Markersubstanz detektiert wurde, die das Hormonrezeptorprotein in Abwesenheit der Testsubstanz markiert, dabei Bestimmen, ob die Testsubstanz eine Substanz ist, die in der Lage ist, mit dem Hormonrezeptorprotein auf der Basis des Unterschieds in beiden detektierten Merkmalen zu interagieren, wobei der Unterschied aus der Dimerisierung des Komplexes des Hormonrezeptorprotein/Testsubstanz und des Monomers resultiert.

2. In vitro Verfahren zur Untersuchung einer Substanz, die mit einem Hormonrezeptor interagiert, umfassend:
a) Behalten eines Hormonrezeptorproteins, das mit einer Markersubstanz markiert ist, die in der Lage ist, ein optisches Signal zu emittieren und eine Testsubstanz unter vorbestimmten Bedingungen, in denen ein natürlicher Ligand mit dem Rezeptorprotein, das mit der Markersubstanz markiert ist, binden kann, und in denen nach der Bindung des natürlichen Liganden, das Hormonreptorprotein ein Dimer eines Komplexes des Hormonrezeptorprotein/Liganden bilden kann, und dann wird es dem Komplex des Hormonrezeptorprotein/Liganden ermöglicht, an eine spezifische Nukleotidsequenz zu binden, wobdurch eine Veränderung in einer detektierbaren Eigenschaft des optischen Signals induziert wird, das von der Markersubstanz, mit der das Hormonrezeptorprotein markiert ist, emittiert wird;
b) Detektieren des optischen Signals, das von der Markersubstanz unter den vorbestimmten Bedingungen emittiert wird; und
c) Vergleichen der Merkmale des optischen Signals, das in Schritt b) detektiert wurde, mit denjenigen des optischen Signals, das von der Markersubstanz detektiert wurde, die das Hormonrezeptorprotein in Abwesenheit der Testsubstanz markiert, dabei Bestimmen, ob die Testsubstanz eine Substanz ist, die in der Lage ist, mit dem Hormonrezeptorprotein zu interagieren, auf der Basis des Unterschieds in beiden detektierten Merkmalen, wobei der Unterschied aus der Dimerisierung des Komplexes des Hormonrezeptorprotein/Testsubstanz und an die spezifische Nucleotidsequenz resultiert.

3. Verfahren gemäß Anspruch 1 oder 2, **dadurch gekennzeichnet, dass** das mit der Markersubstanz markierte Hormonrezeptorprotein, das in der Lage ist, ein optisches Signal zu emittieren, ein Fusionsprotein ist, das ein Hormonrezeptorprotein umfasst, und die Markersubstanz in der Lage ist, ein optisches Signal zu emittieren.

4. Verfahren gemäß Anspruch 1, **dadurch gekennzeichnet, dass** die Veränderung eines detektierbaren Merkmals eines optischen Signals eine Veränderung der Wellenlänge des optischen Signals ist.

5. Verfahren gemäß Anspruch 2, **dadurch gekennzeichnet, dass** die Veränderung eines detektierbaren Merkmals eines optischen Signals eine Veränderung der intrazellulären Verteilung des optischen Signals ist.

6. Verfahren gemäß einem der Ansprüche 1 bis 3 und 5, **dadurch gekennzeichnet, dass** die Veränderung eines detektierbaren Merkmals eines optischen Signals durch Fluoreszenzkorrelationsspektroskopie (FCS) detektiert wird.

7. Verfahren gemäß einem der Ansprüche 1 bis 3, **dadurch gekennzeichnet, dass** es einen Schritt umfasst, in dem eine Testsubstanz mit einer Zelle in Kontakt gebracht wird, die in einem Zustand ist, in dem sie das mit der Markersubstanz, die ein optisches Signal emittieren kann, markierte Hormonrezeptorprotein exprimieren kann.

8. Verfahren gemäß Anspruch 7, **dadurch gekennzeichnet, dass** der Detektierungsschritt den Schritt der Detektierung eines optischen Signals, das von der Markersubstanz in eine Zelle emittiert wird, umfasst.

9. Verfahren gemäß Anspruch 8, **dadurch gekennzeichnet, dass** das optische Signal bei dem Detektierungsschritt dadurch detektiert wird, dass auf den inneren Bereich einer Zelle fokusiert wird.

10. Verfahren gemäß einem der Ansprüche 1 bis 3, **dadurch gekennzeichnet, dass** es einen Schritt des Mischens und Umsetzens der Testsubstanz mit dem Hormonrezeptorprotein umfasst, das mit der Markersubstanz markiert ist, die in der Lage ist, das optische Signal zu emittieren, in einer Flüssigkeit, worin das Protein aus einer Zelle, die sich selbst exprimiert, suspendiert wird.

11. Verfahren gemäß Anspruch 7, **dadurch gekennzeichnet, dass** es ferner den Schritt des Screenens einer Zelle, die zur FCS-Detektion geeignet ist, aus Zellen, die das Hormonrezeptorprotein exprimieren, das mit der Markersubstanz markiert ist, die in der Lage ist, ein optisches Signal zu emittieren, umfasst.

12. Verfahren gemäß Anspruch 11, **dadurch gekennzeichnet, dass** es einen Schritt des Schreenens einer Zelle, die zur FCS-Detektion aus Zellen geeignet ist, die das mit der Markersubstanz markierte Hormonrezeptorenprotein zu exprimieren, das in der Lage ist, ein optisches Signal zu emittieren, basierend auf der Intensität eines optischen Signals, das aus der fluoreszenten Substanz emittiert wird.

13. Verfahren gemäß Anspruch 11, **dadurch gekennzeichnet, dass** es einen Schritt des Schreenens nach einer Zelle, deren Intensität eines optischen Signals per Zelle in den Bereich von 0,2-2400 fällt, umfasst, vorausgesetzt, dass die Intensität einer negativen Kontrolle (eine Zelle, die kein optisches Signal enthält) auf 0,1 festgelegt ist, aus Zellen, die das Hormonrezeptorprotein exprimieren, das mit der Markersubstanz, die in der Lage ist, ein optisches Signal zu emittieren, markiert ist.

14. Verfahren gemäß einem der Ansprüche 11 bis 13, **dadurch gekennzeichnet, dass** es einen Schritt des Schreenens nach einer Zelle umfasst, bei dem das markierte Hormonrezeptorprotein seine physiologische Funktion beibehält, aus Zellen, die das Hormonrezeptorprotein exprimieren, das mit der Markersubstanz, die in der Lage ist, ein optisches Signal zu emittieren, markiert ist.

15. Verfahren gemäß einem der Ansprüche 11 bis 14, **dadurch gekennzeichnet, dass** eine Substanz, die die FSC-Messung hemmt, aus einer Zellkulturlösung entfernt wird.

16. Verfahren gemäß einem der Ansprüche 1 bis 15, **dadurch gekennzeichnet, dass** es einen Schritt des In-Kontakt-Bringens der Testsubstanz mit einem Extrakt umfasst, das aus einem lebenden Körper erhalten wurde, und eine arzneimittelmetabolische Aktivität aufweist.

17. Verfahren gemäß einem der Ansprüche 1 bis 16, bei dem das Hormonrezeptorprotein ein Estrogenrezeptorprotein ist.

18. Verfahren gemäß einem der Ansprüche 1 bis 17, wobei das Hormonrezeptorprotein ein humaner Estrogenrezeptor β ist.

19. Verfahren gemäß einem der Ansprüche 1 bis 18, wobei die Markersubstanz ein Fluoreszenzprotein ist.

## Revendications

1. Procédé *in vitro* visant à tester une substance interagissant avec un récepteur hormonal **caractérisé en ce qu'**il comprend :
(a) maintenir une protéine de récepteur hormonal marquée avec une substance de marquage susceptible d'émettre un signal optique et une substance d'essai dans des conditions prédéterminées où un ligand naturel peut se fixer à la protéine formant récepteur marquée avec la substance de marquage et où, après fixation du ligand naturel, la protéine formant récepteur hormonal peut former un dimère d'un complexe protéine formant récepteur hormonal/ligand, induisant ainsi une modification d'une caractéristique détectable du signal optique émis par la substance de marquage marquant la protéine formant récepteur hormonal ;
(b) détecter le signal optique émis par la substance de marquage dans les conditions prédéterminées ; et
(c) comparer la caractéristique du signal optique détecté à l'étape (b) à celle du signal optique détecté à partir de la substance de marquage marquant la protéine formant récepteur hormonal en l'absence de la substance d'essai, déterminant ainsi si la substance d'essai est ou non une substance susceptible d'interagir avec la protéine formant récepteur hormonal d'après la différence entre les deux caractéristiques détectées, la différence résultant de la dimérisation du complexe protéine formant récepteur hormonal/substance d'essai et du monomère.

2. Procédé *in vitro* visant à tester une substance interagissant avec un récepteur hormonal qui comprend :
(a) maintenir une protéine formant récepteur hormonal marquée avec une substance de marquage susceptible d'émettre un signal optique et une substance d'essai dans des conditions prédéterminées où un ligand naturel peut se fixer à la protéine réceptrice marquée avec la substance de marquage et où, après fixation du ligand naturel, la protéine formant récepteur hormonal peut former un dimère d'un complexe protéine formant récepteur hormonal/ligand, puis le complexe protéine formant récepteur hormonal/ligand peut se fixer à une séquence nucléotidique spécifique, induisant ainsi une modification d'une caractéristique détectable du signal optique émis par la substance de marquage marquant la protéine formant récepteur hormonal ;
(b) détecter le signal optique émis par la substance de marquage dans les conditions prédéterminées ; et
(c) comparer la caractéristique du signal optique détecté à l'étape (b) à celle du signal optique détecté à partir de la substance de marquage marquant la protéine formant récepteur hormonal en l'absence de la substance d'essai, déterminant ainsi si la substance d'essai est ou non une substance susceptible d'interagir avec la protéine formant récepteur hormonal d'après la différence entre les deux caractéristiques détectées, la différence résultant de la fixation du dimère du complexe protéine formant récepteur hormonal/substance d'essai à la séquence nucléotidique spécifique.

3. Procédé selon la revendication 1 ou 2, **caractérisé en ce que** la protéine formant récepteur hormonal marquée avec la substance de marquage susceptible d'émettre un signal optique est une protéine de fusion comprenant une protéine formant récepteur hormonal et la substance de marquage susceptible d'émettre un signal optique.

4. Procédé selon la revendication 1, **caractérisé en ce que** la modification d'une caractéristique détectable du signal optique est une modification de la longueur d'onde du signal optique.

5. Procédé selon la revendication 2, **caractérisé en ce que** la modification d'une caractéristique détectable du signal optique est une modification de la distribution intracellulaire du signal optique.

6. Procédé selon l'une quelconque des revendications 1 à 3 et 5, **caractérisé en ce que** la modification d'une caractéristique détectable du signal optique est détectée par spectroscopie de corrélation de fluorescence (FCS).

7. Procédé selon l'une quelconque des revendications 1 à 3, **caractérisé en ce qu'**il comprend une étape consistant à mettre une substance d'essai en contact avec une cellule, qui est dans un état d'expression de la protéine formant récepteur hormonal marquée avec la substance de marquage susceptible d'émettre un signal optique.

8. Procédé selon la revendication 7, **caractérisé en ce que** l'étape de détection comprend l'étape de détection d'un signal optique émis par la substance de marquage dans une cellule.

9. Procédé selon la revendication 8, **caractérisé en ce que**, à l'étape de détection, le signal optique est détecté par focalisation dans une région interne d'une cellule.

10. Procédé selon l'une quelconque des revendications 1 à 3, **caractérisé en ce qu'**il comprend une étape consistant à mélanger et faire réagir la substance d'essai avec la protéine formant récepteur hormonal qui est marquée avec la substance de marquage susceptible d'émettre le signal optique dans un liquide où la protéine extraite d'une cellule exprimant celle-ci est mise en suspension.

11. Procédé selon la revendication 7, **caractérisé en ce qu'**il comprend en outre une étape consistant à cribler une cellule adaptée à une détection par FCS à partir de cellules exprimant la protéine formant récepteur hormonal marquée avec la substance de marquage susceptible d'émettre un signal optique.

12. Procédé selon la revendication 11, **caractérisé en ce qu'**il comprend une étape consistant à cribler une cellule susceptible d'une détection par FCS à partir de cellules exprimant la protéine formant récepteur hormonal marquée avec la substance de marquage susceptible d'émettre un signal optique d'après l'intensité du signal optique émis par la substance fluorescente.

13. Procédé selon la revendication 11, **caractérisé en ce qu'**il comprend une étape consistant à cribler une cellule dont l'intensité du signal optique par cellule se situe dans une fourchette de 0,2-240, à condition que l'intensité d'un contrôle négatif (une cellule ne contenant pas de signal optique) soit réglée sur 0,1, à partir de cellules exprimant la protéine formant récepteur hormonal marquée avec la substance de marquage susceptible d'émettre un signal optique.

14. Procédé selon l'une quelconque des revendications 11 à 13, **caractérisé en ce qu'**il comprend une étape consistant à cribler une cellule où la protéine formant récepteur hormonal marquée conserve sa fonction physiologique, à partir de cellules exprimant la protéine formant récepteur hormonal marquée avec la substance de marquage susceptible d'émettre un signal optique.

15. Procédé selon l'une quelconque des revendications 11 à 14, **caractérisé en ce qu'**une substance inhibant la mesure par FCS est retirée d'une solution de culture cellulaire.

16. Procédé selon l'une quelconque des revendications 1 à 15, **caractérisé en ce qu'**il comprend une étape consistant à mettre la substance d'essai en contact avec un extrait qui a été obtenu à partir d'un corps vivant et possède une activité métabolique envers les médicaments.

17. Procédé selon l'une quelconque des revendications 1 à 16, dans lequel la protéine formant récepteur hormonal est une protéine formant récepteur aux oestrogènes.

18. Procédé selon l'une quelconque des revendications 1 à 17, dans lequel la protéine formant récepteur hormonal est le récepteur β humain aux oestrogènes.

19. Procédé selon l'une quelconque des revendications 1 à 18, dans lequel la substance de marquage est une protéine fluorescente.
